(19) Europäisches Patentamt / European Patent Office / Office européen des brevets

(11) **EP 4 091 591 B1**

(12) **EUROPEAN PATENT SPECIFICATION**

(45) Date of publication and mention
of the grant of the patent:
**04.09.2024 Bulletin 2024/36**

(21) Application number: **21175209.2**

(22) Date of filing: **21.05.2021**

(51) International Patent Classification (IPC):
***A61F 13/02*** *(2024.01)* ***B32B 43/00*** *(2006.01)*
***B29C 65/00*** *(2006.01)*

(52) Cooperative Patent Classification (CPC):
**A61F 13/0276;** B32B 37/226; B32B 2305/34;
B32B 2535/00

(54) **APPARATUS FOR LAMINATING AT LEAST ONE LAYER ONTO A WOUND CARE ARTICLE STRUCTURE**

VORRICHTUNG ZUM AUFLAMINIEREN MINDESTENS EINER LAGE AUF EINE WUNDPFLEGEARTIKELSTRUKTUR

APPAREIL DE STRATIFICATION D'AU MOINS UNE COUCHE SUR UNE STRUCTURE D'ARTICLE DE SOIN DES PLAIES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**23.11.2022 Bulletin 2022/47**

(73) Proprietor: **Paul Hartmann AG**
**89522 Heidenheim (DE)**

(72) Inventors:
• **RÖHRL, Wolfgang**
**89522 Heidenheim (DE)**
• **RODRIGUEZ, Marc**
**08302 Mataró (ES)**

(74) Representative: **Ter Meer Steinmeister & Partner**
**Patentanwälte mbB**
**Nymphenburger Straße 4**
**80335 München (DE)**

(56) References cited:
**US-A1- 2007 113 972**

## Description

## FIELD

Embodiments of the present disclosure relate to an apparatus and a method for laminating at least one layer onto a wound care article structure. Embodiments of the present particularly relate to the manufacture of wound care articles.

## BACKGROUND

Wound care articles, such as dressings, are sterile articles that can be applied to a wound to promote healing and protect the wound from further damage. A variety of wound care articles are known that can promote healing of various types of wounds, such as cuts, abrasions, and blisters of skin tissue. Some wound care articles consist of a number of different layers which can be laminated on top of each other in a continuous manner. In particular, an article structure may move along a production line as some layers are added to the article structure and other layers, such as temporary protective layers, are removed. At the end of the production line, the finished product may then be present.

Wound care articles are mass-produced items that are manufactured in large quantities. Therefore, the efficiency and production speed of a manufacturing system is critical to increasing the yield of the manufacturing system. However, the addition and removal of layers can limit the production speed and thus the yield.

In view of the above, new manufacturing systems for wound care articles that overcome at least some of the problems in the art are beneficial.
US 2017/0113972 A1 relates to a method of manufacturing a flexible laminate substrate, wherein a metal foil is bonded to at least one surface of a heat-resistant adhesive film. The method comprises a step wherein the heat-resistant adhesive film and the metal foil are thermally laminated between a pair of metal rolls via a protective film, thereby forming a laminate wherein the heat-resistant adhesive film, the metal foil, and the protective film are bonded together. Furthermore, nip rolls are provided for thermally laminating the metal foil and the heat-resistant adhesive film through the protective film. After the laminate has passed the nip rolls, the protective film is being delaminated from the laminate.

## SUMMARY

In light of the above, an apparatus and a method for laminating at least one layer onto a wound care article structure are provided.

It is an object of the present disclosure to improve the manufacturing of wound care article structures. It is another object of the present disclosure to increase a manufacturing speed and a yield of a manufacturing system for wound care articles. Furthermore, it is an object to keep a required quality of the manufactured article structures and the final wound care article, when driving the manufacturing system with higher speed or to improve the quality of the final wound care article in particular for wound pads having a high height difference to the carrier, i.e. being thick or having an irregular surface.

The objects are solved by the features of the independent claims. Preferred embodiments are defined in the dependent claims. Further aspects, benefits, and features of the present disclosure are apparent from the claims, the description, and the accompanying drawings.

An apparatus for laminating at least one layer onto a wound care article structure is disclosed in claim 1.

The delamination arrangement might be placed at a different location in the process line.

According to some embodiments, which can be combined with other embodiments described herein, the delamination arrangement includes one or more delamination rolls and/or one or more delamination blades.

Furthermore, the pressure arrangement can be arranged also behind the delamination arrangement or there might be a further pressure arrangement behind the delamination arrangement.

According to some embodiments, which can be combined with other embodiments described herein, the at least one layer is a multilayered structure, e.g. Acrysil™.

Preferably, the pressure arrangement is configured to apply the pressure to the multilayered structure to press an adhesive layer, such as an acrylic layer, of the multilayered structure against another adhesive layer, such as another acrylic layer, of the wound care article structure.

The multilayered structure might be a highly flexible and/or thin foil or layer. It might be also called wound contact layer which is fixed on the wound care article structure. The wound care article structure might include a carrier having the wound pads attached thereto. The carrier might be called also carrier layer or back sheet.

Preferably, the at least one layer may be a multilayered structure.

Preferably the pressure arrangement may apply the pressure to the multilayered structure to tightly press the multilayered structure with its a first layer, such as a first adhesive layer, of the multilayered structure against the wound care article structure, in particular against a second layer, such as a second adhesive layer, which might represent the top layer of the wound care article structure. However, here it might be also sufficient if the first layer of the multilayered structure is adhesive, since it is pressed by the pressure arrangement to the wound care article structure and thus sufficiently attached or glued onto this carrier or backsheet.

Preferably, the first and second layers are pressure sensitive adhesive layers, preferably adhesive acrylic layers.

According to some embodiments, which can be combined with other embodiments described herein, the first lamination arrangement is further configured for delaminating at least one protective layer off the multilayered structure before the multilayered structure is laminated onto the wound care article structure.

According to some embodiments, which can be combined with other embodiments described herein, the support surface of the conveyor includes a first side configured for supporting the wound care article structure and a second side opposite the first side.

Preferably, at least one first pressure roll of the one or more pressure rolls is located at the first side of the support surface.

According to some embodiments, which can be combined with other embodiments described herein, the one or more pressure rolls are a plurality of pressure rolls.

Preferably, at least one second pressure roll of the plurality of pressure rolls is located at the second side of the support surface.

The one or more pressure rolls of the pressure arrangement are adjustable in their distance to the wound care article structure or in their distance to the carrier. Thus, the pressure which acts onto the layers may be set or adjusted. The distance between the surface of the one or more pressure rolls and the layer to be pressed onto the carrier or onto the wound care article structure might depend on the or more materials used for the carrier or onto the wound care article structure and may depend on the structure of the wound pad. Thus, the distance in the area surrounding the wound pad might be set smaller than in the area of the wound pad. Thus, the applied pressure might be adjusted and might higher in the area surrounding the wound pad.

Preferably, the support surface and the wound care article structure are interposed between the at least one first pressure roll and the at least one second pressure roll.

According to some embodiments, which can be combined with other embodiments described herein, the support surface is a flexible support surface. A surface elasticity of the flexible support surface may allow uniform pressure distribution over the article cross-section.

According to some embodiments, which can be combined with other embodiments described herein, the support surface is a substantially inflexible support surface.

According to some embodiments, which can be combined with other embodiments described herein, the one or more pressure rolls are located only at the first side of the support surface.

According to some embodiments, which can be combined with other embodiments described herein, the at least one first pressure roll includes at least one constant diameter roll having a substantially constant diameter.

According to some embodiments, which can be combined with other embodiments described herein, the at least one first pressure roll includes at least one variable diameter roll having a variable diameter. In particular, the diameter of the at least one variable diameter roll may vary in a direction substantially parallel to a rotational axis of the at least one variable diameter roll.

According to some embodiments, which can be combined with other embodiments described herein, the at least one variable diameter roll has at least one protruding portion and at least one recessed portion.

Preferably, a diameter of the at least one protruding portion is larger than a diameter of the at least one recessed portion.

According to some embodiments, which can be combined with other embodiments described herein, a width of the at least one recessed portion in a direction substantially parallel to a rotational axis of the at least one variable diameter roll is equal to or larger than a width of an elevated pad portion (e.g. a wound pad) of the wound care article structure. Thus, a wound pad having a large thickness and/or having a surface being irregular or a 3D-structure can be tightly fixed to the carrier by using the recessed part of the pressure rolls.

According to some embodiments, which can be combined with other embodiments described herein, a depth of the at least one recessed portion in a direction substantially perpendicular to the rotational axis of the at least one variable diameter roll is less, equal to, or larger than a height of the elevated pad portion of the wound care article structure. The depth of the at least one recessed portion may be defined as a difference between the diameter of the at least one protruding portion and a diameter of the at least one recessed portion. So, depending on the depth of the recessed portion or distance between the surface in the recessed portion and the multilayered structure being pressed onto the carrier, there might be a further pressure applied also in the area of the wound pad if the distance is smaller than the height of the wound pad. Otherwise, it might be possible not to apply any pressure in the area of the wound pad. Finally, the applied pressure in the area of the wound pad might be set differently than in the area surrounding or outside the wound pad.

According to some embodiments, which can be combined with other embodiments described herein an area of the at least one recessed portion may be adapted to the form of the elevated pad portion of the wound care article structure. So, the recessed portion of the one or more pressure rolls might be rectangular, circular or irregular and/or might be adapted to the form of the wound pad.

So, by having one or more pressure rolls having one or more recessed portions, it might be possible to have areas in which no additional pressure is applied at all to the multilayered structure when being pressed onto the wound care article structure.

According to some embodiments, which can be combined with other embodiments described herein, the pressure arrangement includes the at least one variable diameter roll and at least one constant roll arranged in series along a transport path of the wound care article structure.

However, it be also possible to provide several first pressure rolls arranged sequentially and having different diameters or different distances to press different portions of the multilayered structure onto the wound care article structure. So, a first roll being thicker in diameter might be used for fixing the portions of the multilayered structure to the carrier, e.g. directly to the backsheet or to second layer of the carrier material. So, this first roll might press the areas excluding the wound pad. Another roll having a smaller diameter than the first might be used to fix or press only the portion of the wound pad. Thus, different lamination levels might be realized to adapt to the height profile of the wound care article and the structure of the wound pad. Since the multilayered structure is very flexible and a thin material, it is possible to prevent or reduce an inclusion of air between the multilayered structure and the wound care article structure, in particular in the area surrounding the wound pad. Thus, a tight contact between the multilayered structure onto the wound pad is achieved and a tight contact between the multilayered structure surrounding

A method for laminating at least one layer onto a wound care article structure is claimed in claim 14.

Preferably, the delamination force has a first delamination force component in a vertical direction and a second delamination force component in a horizontal direction. The first delamination force component is smaller than a vertical force component generated by the conveyor device and acting on the article structure in a direction opposite to the first delamination force component.

According to some embodiments, which can be combined with other embodiments described herein, a delamination angle defined between a direction or vector of the delamination force and the first delamination force component is 10° or higher and/or less than 90°. For example, the delamination angle is 15° or higher, 20° or higher, 25° or higher, 30° or higher, 35° or higher, 40° or higher, or 45° or higher. In any case, the delamination angle may be less than 90°.

According to some embodiments, which can be combined with other embodiments described herein, the support surface is inclined with respect to the horizontal direction by an inclination angle to provide the first delamination force component that is smaller than the vertical force component generated by the conveyor device.

Preferably, the inclination angle may be 45° or less and more than 0°. For example, the inclination angle may be 40° or less, 35° or less, 30° or less, 25° or less, 20° or less, 15° or less, or 10° or less. In any case, the inclination angle may be greater than 0°. In a particular embodiment, the inclination angle may be about 17°.

According to some embodiments, which can be combined with other embodiments described herein, the conveyor device includes a suction mechanism configured to hold the article structure at the support surface by means of a suction force.

Preferably, the vertical force component of the conveyor device includes a vertical component of the suction force

According to some embodiments, which can be combined with other embodiments described herein, the one or more delamination rolls are two delamination rolls arranged so that the protective layer removed from the article structure is guided between them.

Preferably, the two delamination rolls are arranged above each other in a direction substantially perpendicular to the support surface.

According to some embodiments, which can be combined with other embodiments described herein, the apparatus further includes a speed control arrangement configured to control a moving speed of the delaminated protective layer.

According to some embodiments, which can be combined with other embodiments described herein, the speed control arrangement includes a determination unit having a pair of fixed rolls and a moveable roll arranged in a triangle and configured to guide the delaminated protective layer coming from the conveyor device.

Preferably, the apparatus is configured to perform a control of the speed of the delaminated protective layer based on a position of the moveable roll.

According to some embodiments, which can be combined with other embodiments described herein, the moveable roll is linearly moveable towards the pair of fixed rolls, preferably to a center between the pair of fixed rolls.

In further embodiments, the moveable roll is substantially circularly moveable.

According to some embodiments, which can be combined with other embodiments described herein, the speed control arrangement includes an encoder device configured to determine the position of the moveable roll by detecting a deflection or position of the moveable roll.

According to some embodiments, which can be combined with other embodiments described herein, the speed control arrangement further includes a pair of driven rolls configured to guide and move the delaminated protective layer coming from the determination unit.

Preferably, the pair of driven rolls is configured to control the moving speed of the delaminated protective layer.

Preferably, the pair of driven rolls is a pair of nip rolls.

According to some embodiments, which can be combined with other embodiments described herein, the apparatus further includes a first lamination arrangement configured for laminating a multilayered structure onto the article structure.

Preferably, the first lamination arrangement is further configured for delaminating at least one protective layer off the multilayered structure before the multilayered structure is laminated onto the article structure.

According to some embodiments, which can be combined with other embodiments described herein, the apparatus further includes a second lamination arrangement configured for laminating at least one layer onto the article structure after the protective layer has been delaminated off the article structure by means of the delamination arrangement.

Preferably, the second lamination arrangement includes one or more lamination rolls.

According to some embodiments, which can be combined with other embodiments described herein, the apparatus includes at least one separation device configured to separate, e.g. cut, the article structure into a plurality of articles.

Preferably, the articles are wound care articles, such as wound dressings.

According to some embodiments, which can be combined with other embodiments described herein, the conveyor device is a belt conveyor.

Preferably, the article structure may include a nonwoven material which is preferably used a carrier or support material.

The nonwoven material may be fully coated with an acrylic coating. At least one or several wound pads may be attached on the acrylic coating. The wound pads may have a predetermined distance in y and/or x direction to the next wound pad. These components may form the article structure described above.

This article structure may be transported on the conveyor in a continuous band form having wound pads being spaced apart from each other on the nonwoven material in a row.

The article structure may also have sheet form having several rows and columns of wound pads attached thereon.

The article structure may by coated with the multilayered structure, e.g. Acrysil ™.

The multilayered structure may have at least one single center layer, e.g. a PU layer being covered on one side facing the article structure with an acrylic coating. Thereby the acrylic coating of the multilayered structure and the acrylic coating of the article structure, e.g. on the nonwoven material may form a strong adhesive connection which provides a reliable attachment of the two material layers, i.e. the article structure and the multilayered structure.

As the multilayered structure is separately manufactured it needs to be laminated onto the article structure. The multilayered structure which may be at least one PU layer having on both side surfaces a coating, may be covered on one or both coatings with protection layers. As the protection layers are only attached temporally on the PU layer being coated with an adhesive coating, the protective layer needs to be removed before fixedly attaching the multilayered structure on the article structure. As the surface of the PU layer facing the article structure side and the wound pad side is coated with an acrylic coating, the protection layer is a siliconized paper, which might be easily removed before laminating the PU layer with it exposed acrylic coating to the acrylic coating on the nonwoven material.

The opposing side of the PU layer may be coated with a siliconized coating. To allow an easy removing or delaminating of the protective layer on this side of the multilayered structure, the protective layer is preferably a PE film, which provides sufficient protection during transport and manufacturing, but which might be easily removed from the siliconized coating.

The manufacturing system or apparatus might be supplied with one or more article structure bands being transported in parallel on the conveyor and maybe processed simultaneously by the lamination and delamination stages.

As indicated above, alternatively, it might be possible to provide a larger sheet also as a continuous band or sheet having a two-dimensional arrangement of wound pads thereon. So, there are several wound pads on one sheet of nonwoven material in x and y direction being spaced apart from each other

Thus, the lamination of the multilayered structure including the delamination of the protection layer is performed over several rows of wound pads. Also, the delamination of the other protective layer from the multilayered structure may be made in a larger width, e.g. over the full width of the conveyor. Thus, the protective layer on the multilayered structure in several parallel rows of wound pads might be simultaneously removed or delaminated.

The same applies for the final lamination of the liner, which might be the last station on the conveyor.

Finally, the sheet of finalized plasters or wound dressings might be separated in x and y direction to provide e.g. a plurality of single plasters or wound dressings.

The delamination force has a first delamination force component in a vertical direction and a second delamination force component in a horizontal direction. The first delamination force component is smaller than a vertical force component generated by the conveyor device and acting on the article structure in a direction opposite to the first delamination force component.

In another aspect of the disclosure, an apparatus for removing at least one layer from an article structure is provided. The apparatus includes a conveyor device having a support surface configured for supporting the article structure; and a delamination arrangement including one or more delamination rolls configured for delaminating a protective layer off the article structure by applying a delamination force to the protective layer. The conveyor device having a support surface might be inclined with respect to a horizontal direction. The apparatus includes a speed control arrangement for controlling the speed of the delamination to thereby control the delamination of the protective layer from the article structure.

**BRIEF DESCRIPTION OF THE DRAWINGS**

So that the manner in which the above recited features of the present disclosure can be understood in detail, a more particular description of the disclosure, briefly summarized above, may be had by reference to embodiments. The accompanying drawings relate to embodiments of the disclosure and are described in the following:

FIG. 1A   shows a cross-sectional side view of an article structure according to embodiments described herein,

FIG. 1B   shows a top view of the article structure of FIG. 1A,

FIG. 1C   shows a top view of a further exemplary article structure;

FIG. 2   shows a schematic side view of an apparatus for laminating at least one layer onto an article structure and removing at least one (further) layer from the article structure according to embodiments described herein;

FIG. 3   shows a perspective view of the apparatus of FIG. 2;

FIG. 4A   shows a schematic side view of an apparatus having a pressure arrangement according to embodiments described herein;

FIG. 4B   shows a schematic side view of an apparatus having a pressure arrangement according to further embodiments described herein;

FIG. 5A   shows a schematic side view of an apparatus having a pressure arrangement according to further embodiments described herein;

FIG. 5B   shows a schematic side view of an apparatus having a pressure arrangement according to further embodiments described herein;

FIGs. 6A-D   show front views of various pressure rolls of a pressure arrangement according to embodiments described herein;

FIGs. 7A-D   show front views of various pressure rolls of a pressure arrangement according to embodiments further described herein;

FIG. 8A   shows forces in an apparatus according to embodiments described herein when removing at least one layer from an article structure; and

FIG. 8B   shows forces in an apparatus according to further embodiments described herein when removing at least one layer from an article structure.

## DETAILED DESCRIPTION OF EMBODIMENTS

Reference will now be made in detail to the various embodiments of the disclosure, one or more examples of which are illustrated in the figures. Within the following description of the drawings, the same reference numbers refer to same components. Generally, only the differences with respect to individual embodiments are described. Each example is provided by way of explanation of the disclosure and is not meant as a limitation of the disclosure. Further, features illustrated or described as part of one embodiment can be used on or in conjunction with other embodiments to yield yet a further embodiment. It is intended that the description includes such modifications and variations.

As described above, wound care articles are mass-produced items that are manufactured in large quantities. Therefore, the efficiency and production speed of a manufacturing system is critical to increasing the yield of the manufacturing system. However, the addition and/or removal of the layers can limit the production speed and thus the yield.

The embodiments of the present disclosure overcome the above drawbacks by pressing a layer or multilayered structure that has been laminated onto a wound care article structure against said wound care article structure. Thereby, an adhesion force between the laminated layer or multilayered structure and the wound care article structure can be enhanced so that a subsequent removal of a protective layer does not result in a detachment of the laminated layer or multilayered structure from the wound care article structure. Accordingly, the production speed and thus the yield of a manufacturing system can be increased.

Further embodiments of the present disclosure, which can be combined with the aforementioned aspect, adjust a delamination force used to peel a temporary protective layer from the article structure. In particular, the delamination force is adjusted so that the article structure adheres to the support surface of the conveyor and does not lift off. Accordingly, the production speed and thus the yield of a manufacturing system can be increased.

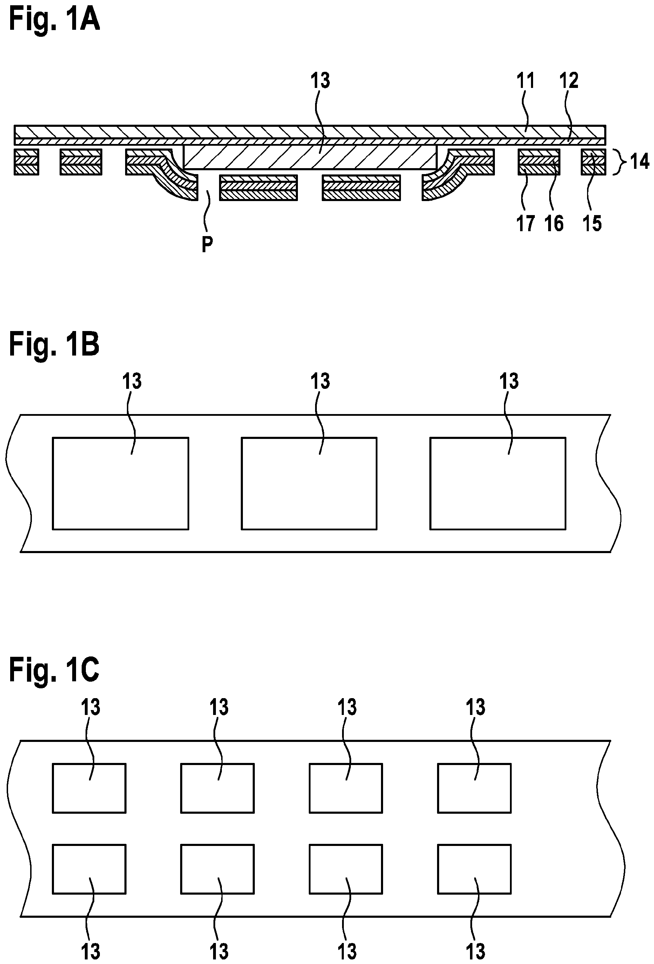

According to some embodiments, which can be combined with other embodiments described herein, the article structure is used to manufacture wound care articles, such as wound dressings. Wound care articles often comprise a multilayered structure laminated one on top of the other. An exemplary wound care article is shown in FIGs. 1A, 1B and 1C.

FIG. 1A shows a cross-sectional view of a wound care article 10 according to embodiments described herein. FIG. 1B shows a top view of a wound care article structure used to manufacture the wound care article 10 of FIG. 1A. FIG. 1C shows a top view of an alternative configuration of the wound care article structure used to manufacture the wound article 10 of FIG. 1A.

The wound care article 10 may include a carrier 11. The carrier 11 may define a side of the wound care article 10 facing away from the wound and forms an outer visible side or covering of the wound care article 10. The term "carrier" as used herein shall particularly embrace flexible carriers. In some implementations, the carrier 11 may include, or consist of, a non-woven fabric or any other suited material, which can carry or support the wound pads 130 and can serve finally as cover layer when the wound is applied to the skin of the body.

The wound care article 10 further includes a wound pad 13 over or on the carrier 11. The wound pad 13 may include an absorbent material that is able to absorb liquids from the wound. The wound pad 13 can include, or consist of, any material suitable for this purpose and can contain, for example, a foam, absorbent fibers, a textile material, a gel or absorbent particles or any combination thereof.

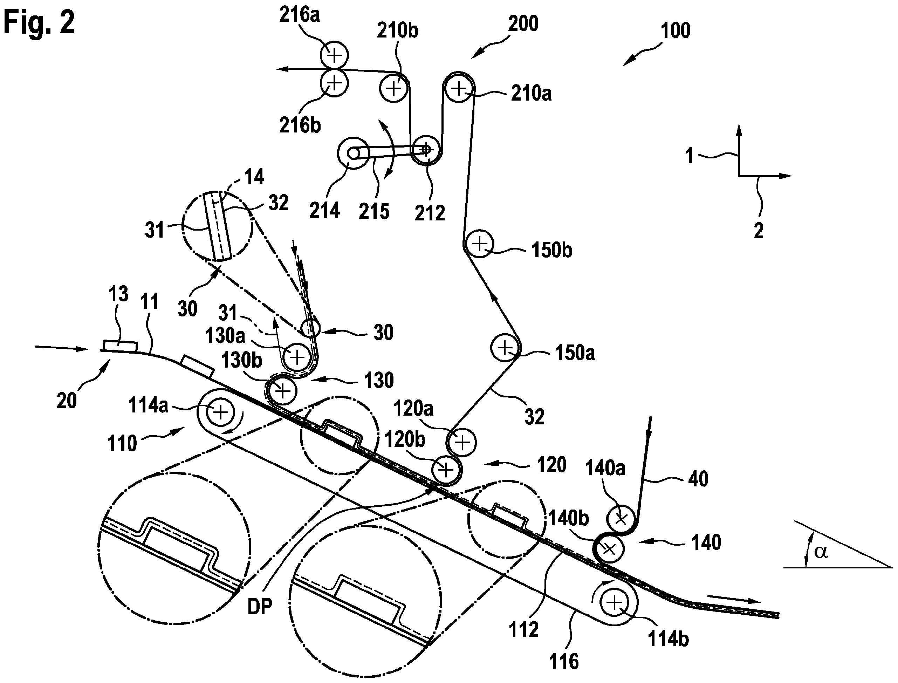

The article structure 20 may be a continuous article structure on which a plurality of wound pads 13 are arranged in series with a predetermined distance therebetween. In the example of FIG. 1B, one single line of wound pads 13 is provided e.g. along the x-direction defined in FIG. 2. In another example, as is shown in FIG. 1C, a two-dimensional matrix of wound pads 13 is arranged in x-direction and y-direction. The sheet of finalized wound care articles, such as plasters, can be separated in x direction and y direction to provide a plurality of individual wound care articles.

In some implementations, the wound care article 10 includes at least one intermediate layer 12 arranged between the carrier 11 and the wound pad 13. The intermediate layer 12 may be configured to attach the wound pad 13 to the carrier 11. For example, the intermediate layer 12 may be an adhesive layer, such as an acrylic layer or acrylic coating.

A multilayered arrangement 14 may be provided over or on the carrier 11 and the wound pad 13. The multilayered arrangement 14 may define a side of the wound care article 10 facing towards the wound. At least one layer of the multilayered arrangement 14 may contact the wound and/or the skin tissue surrounding the wound. The multilayered arrangement 14 may be configured to adhere the wound care article 10 to the wound and/or the skin tissue surrounding the wound.

In some embodiments, the multilayered arrangement 14 may have openings or perforations P such that fluids can pass from the wound into the wound pad 13. The openings or perforations P can be distributed irregularly over the surface of the multilayered arrangement 14 or be arranged in regular patterns.

In the example of FIG. 1A, the multilayered arrangement 14 has three layers. However, the present disclosure is not limited thereto and the multilayered arrangement 14 may have two layers or more than three layers. In other embodiments, the multilayered arrangement 14 can be omitted and a single layer can be provided instead.

The multilayered arrangement 14 may have a first layer 15, a second layer 16 and a third layer 17.

The first layer 15 may be configured to adhere the multilayered arrangement 14 to the wound pad 13 and/or the carrier 11 and/or the intermediate layer 12. For example, the first layer 15 may be an acrylic layer or acrylic coating, in particular an adhesive medical acrylic layer or adhesive medical acrylic coating.

The second layer 16 may be an intermediate layer, such as a polyurethane (PU) layer or PU coating.

The third layer 17 may be a contact layer configured to contact the wound and/or skin surrounding the wound. In particular, the third layer 17 may be configured to adhere the wound care article 10 to the wound and/or the skin. In some embodiments, the third layer 17 may be a silicone coating or silicone layer, such as a soft tack silicone gel coating or layer.

In some embodiments, the multilayered arrangement 14 may be Acrysil ™.

A release liner (not shown) may be provided over the multilayered arrangement 14. The release liner may be removed to expose the third layer 17 before the wound care article 10 is attached to a body.

Typically, when reference is made to the term "over", i.e., one layer being over the other, it is understood that, starting from the carrier, a first layer is deposited over the carrier, and a further layer, laminated after the first layer, is thus over the first layer and over the carrier. In other words, the term "over" is used to define an order of layers, layer stacks, and/or coatings wherein the starting point is the carrier. This is irrespective of whether the wound care article is depicted upside down or not.

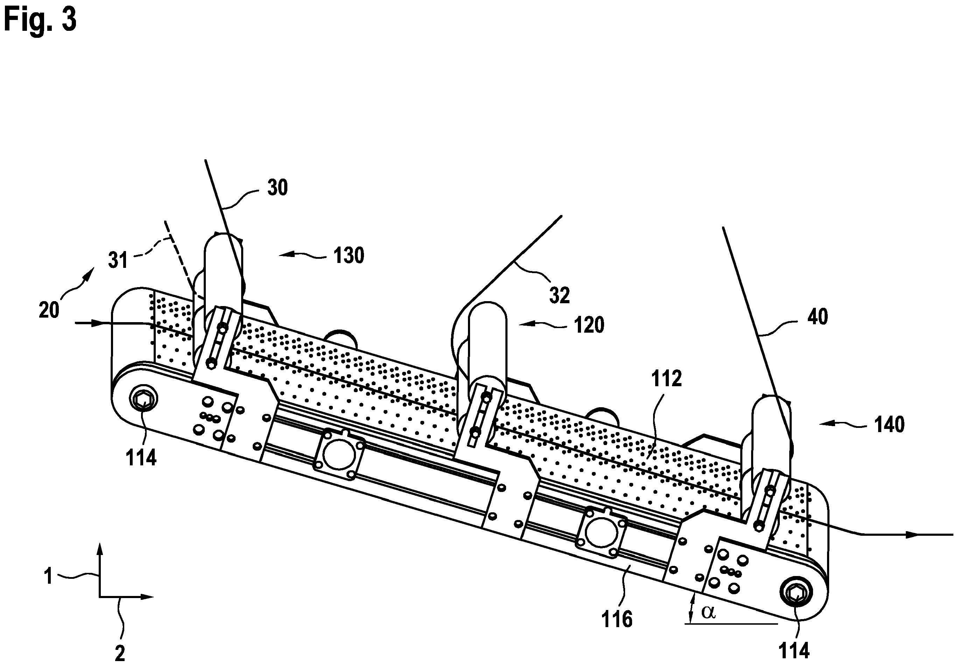

FIG. 2 shows a schematic view of an apparatus 100 for laminating at least one layer onto a wound care article structure 20 and removing at least one layer from the article structure 20 according to embodiments described herein. FIG. 3 shows a perspective view of the apparatus 100 of FIG. 2. The apparatus 100 may be used in the manufacture of wound care articles, such as wound dressings, and may be part of a larger production line. In FIGs. 2 and 3 the pressure arrangement are not illustrated.

The apparatus 100 includes a support surface 112 configured for supporting the article structure 20. The support surface 112 may be an essentially flat support surface of the conveyor device 110. The conveyor device 110 may be configured for moving the article structure 20 along a transport path, in particular along an essentially linear transport path.

In some embodiments, the article structure 20 may be a continuous structure which is cut at the end of the manufacturing process into a plurality of individual wound care articles. In particular, the article structure 20 may move along a production line including the apparatus 100 as some layers are added to the article structure and other layers, such as temporary protective layers, are removed. At the end of the production line, the finished product may then be present. Accordingly, it should be understood that the composition of the article structure 20 changes in the course of production, because layers are removed while others are added.

In some embodiments, the conveyor device 110 may include a belt 116 which provides the support surface 112. In this case, the conveyor device 110 may be a belt conveyor. The belt 116 may be movable to transport the article structure 20 along the transport path. In some implementations, the conveyor device 110 can include one or more conveyor rolls, such as a first conveyor roll 114a and a second conveyor 114b, configured to move the belt 116. The conveyor rolls may be rotatable around respective rotational axes to move the belt 116 for a transportation of the article structure 20 along the transport path. The rotational axes of the conveyor rolls may be substantially horizontally oriented rotational axis.

As used in the present disclosure, the horizontal direction is the direction perpendicular to the vertical direction. The vertical direction is parallel to gravity.

According to some embodiments, which can be combined with other embodiments described herein, the conveyor device 110 includes a suction mechanism (not shown) configured to hold the article structure 20 at the support surface 112 by means of a suction force. For example, a plurality of vacuum ports may be formed in the support surface 112 to apply a vacuum or underpressure on a side of the article structure 20 to adhere the article structure 20 to the support surface 112. In some implementations, the suction force is created by use of a vacuum source (not shown) coupled to the vacuum ports in the support surface 112.

As is shown in FIG. 2, the article structure 20 enters the apparatus 100 at a first side of the apparatus 100, is processed and exits the apparatus 100 at a second side opposite the first side with respect to the transport path of the article structure 20. The processing of the article structure 20 is explained in the following.

The article structure 20 entering the apparatus 100 may already include the carrier 11 and the wound pads 13 of the wound care article shown in FIGs. 1A-C. The carrier 11 and the wound pads 13 may be attached to each other by means of an intermediate layer (not shown), such as an acrylic layer or acrylic coating.

The apparatus 100 may be configured to laminate at least one layer, such as a multilayered structure 30, on the article structure 20 using a first lamination arrangement 130. The multilayered structure 30 may include the multilayered structure 14 shown in FIG. 1A and may initially have protective layers 31, 32 on each side thereof. In particular, the multilayered structure 30 may have a first protective layer 31 on a first side thereof and a second protective layer 32 on a second side thereof. The first side of the multilayered structure 30 may be a side facing towards the article structure 20, in particular the carrier 11.

The first lamination arrangement 130 may be configured to remove at least one protective layer from at least one of the sides of the multilayered structure 30 before the multilayered structure 30 is laminated onto the article structure 20. For example, the first lamination arrangement 130 may be configured to remove the first protective layer 31 from the first side of the multilayered structure 30 which faces the article structure 20 before the multilayered structure 30 is laminated onto the article structure 20.

Referring to FIG. 1A, the protective layer 31 may cover and protect the first layer 15, such as an acrylic layer or acrylic coating, used to adhere the multilayered structure 30 to the article structure 20. After the protective layer 31 has been removed from the multilayered structure 30, the first lamination arrangement 130 may then apply the multilayered structure 30 to the article structure 20. The protective layer 31 may be a siliconized protection layer or siliconized paper.

According to some embodiments, the first lamination arrangement 130 may include two or more rolls, such as a first roll 130a and a second roll 130b, configured to laminate the at least one layer, such as the multilayered structure 30, onto the article structure 20. Optionally, the two or more rolls may be configured to remove the at least one protective layer, such as the first protective layer 31, from the multilayered structure 30 before the multilayered structure 30 is laminated onto the article structure 20.

The first roll 130a might be designated as bottom roller which could have a surface of rubber and/or foam to adapt to the thickness of the laminating surface of the article structure. The second roll 130b might have a different surface to reliable transfer the delaminated protection layer.

For example, the first roll 130a and the second roll 130b may define a space or gap therebetween. The at least one protective layer, such as the first protective layer 31, may be removed from the multilayered structure 30 at a position behind the first roll 130a, wherein it is then wound around the first roll 130a to pass through the gap between the first roll 130a and the second roll 130b.

Preferably, the first roll 130a and the second roll 130b are arranged above each other in a direction substantially perpendicular to the support surface 112. The term "substantially perpendicular" relates to a substantially perpendicular orientation of a line on which the first roll 130a and the second roll 130b are arranged, wherein a deviation of a few degrees, e.g. up to 5° or even up to 10°, from an exact perpendicular orientation is still considered as "substantially perpendicular".

Each of the two or more rolls, such as the first roll 130a and the second roll 130b, may have a respective rotational axis. The rotational axes of the two or more rolls may be substantially parallel to each other. The term "substantially parallel" relates to a substantially parallel orientation of the rotational axes, wherein a deviation of a few degrees, e.g. up to 10° or even up to 15°, from an exact parallel orientation is still considered "substantially parallel". In some embodiments, the rotational axes of the two or more rolls may be substantially horizontally oriented rotational axes.

In some implementations, the two or more rolls may be cylindrical rolls. The term "cylinder" can be understood as commonly accepted as having a circular bottom shape and a circular upper shape and a curved surface area or shell connecting the upper circle and the lower circle. A cylinder axis of the cylindrical roll may define, or be, the rotational axis of this roll.

Typically, the rotational axes of the two or more rolls of the first lamination arrangement 130, such as the rotational axes of the first roll 130a and the second roll 130b, are substantially parallel to the support surface 112.

Accordingly, a modified article structure having the multilayered structure 30 arranged on the article structure 30 is provided, which moves as a continuous band along the transport path and reaches a delamination arrangement 120. The delamination arrangement 120 is configured for delaminating a protective layer off the article structure 20, such as the second protective layer 32 which was laminated together with the multilayered structure 30. The second protective layer 32 may cover the third layer of the previously laminated multilayered structure 30, such as a silicone coating or silicone layer.

The delamination arrangement 120 includes one or more delamination rolls, such as a first delamination roll 120a and a second delamination roll 120b, configured for delaminating the protective layer 32 off the article structure 20 by applying a delamination force to the protective layer 32.

In an exemplary embodiment, the delamination force has a first delamination force component in a vertical direction 1 and a second delamination force component in a horizontal direction 2. The first delamination force component may be smaller than a vertical force component generated by the conveyor device 110 and acting on the article structure 20 in a direction opposite to the first delamination force component. It is to be understood that these forces and force components act on a delamination point DP, at which the protective layer 32 detaches from the article structure 20.

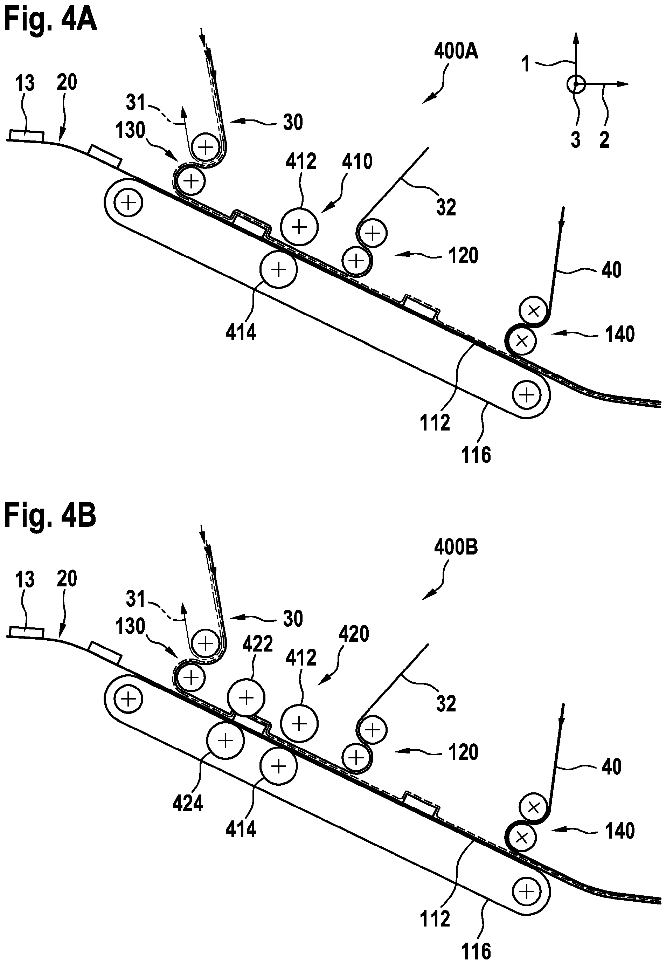

Accordingly, the delamination force generated by the delamination arrangement 120 is adjusted so that the article structure 20 adheres to the support surface 112 of the conveyor 110 and does not lift off. Furthermore, it has to be assured that the multilayered structure 14 laminated onto the article structure 20 is not lifted off during the delamination process of the protective layer 32. Therefore, the production speed and thus the yield of a manufacturing system can be increased. The delamination force and its adjustment are explained in detail with reference to FIGs. 4A and 4B and reference is made to this description for details.

According to some embodiments, which can be combined with other embodiments described herein, the support surface 112 is inclined with respect to the horizontal direction 2 by an inclination angle $\alpha$ to provide the adjustment of the delamination force generated by the delamination arrangement 120. In particular, the inclination angle $\alpha$ may be 45° or less and more than 0°. For example, the inclination angle $\alpha$ may be 40° or less, 35° or less, 30° or less, 25° or less, 20° or less, 15° or less, or 10° or less. In any case, the inclination angle $\alpha$ may

be greater than 0°. In a particular embodiment, the inclination angle $\alpha$ may be about 17°. However, the embodiments of the present disclosure are not limited thereto and the support surface 112 may not be inclined with respect to the horizontal direction 2. In other words, the support surface 112 may be an essentially horizontal support surface (i.e., $\alpha=0$).

In some embodiments, the one or more delamination rolls are two delamination rolls, such as the first delamination roll 120a and the second delamination roll 120b. The first delamination roll 120a and the second delamination roll 120b can be arranged so that the protective layer 32 removed from the article structure 20 can be guided between them away from the article structure 20.

For example, the first delamination roll 120a and the second delamination roll 120b may define a space or gap therebetween through which the protective layer 32 removed from the article structure 20 passes.

The first delamination roll 120a might be designated as bottom roller which could have a surface of rubber and/or foam to adapt to the thickness of the laminating surface of the article structure.

Preferably, the first delamination roll 120a and the second delamination roll 120b are arranged above each other in a direction substantially perpendicular to the support surface 112. The term "substantially perpendicular" relates to a substantially perpendicular orientation of a line on which the first delamination roll 120a and the second delamination roll 120b are arranged, wherein a deviation of a few degrees, e.g. up to 5° or even up to 10°, from an exact perpendicular orientation is still considered as "substantially perpendicular".

Each of the one or more delamination rolls, such as the first delamination roll 120a and the second delamination roll 120b, may have a respective rotational axis. The rotational axes of multiple delamination rolls may be substantially parallel to each other.

In some implementations, the one or more delamination rolls may be cylindrical delamination rolls. A cylinder axis of the cylindrical delamination roll may define, or be, the rotational axis of this delamination roll.

Typically, the rotational axes of the one or more delamination rolls, such as the rotational axes of the first delamination roll 120a and the second delamination roll 120b, are substantially parallel to the support surface 112.

According to some embodiments, which can be combined with other embodiments described herein, the apparatus 100 further includes a speed control arrangement 200 configured to control a moving speed of the delaminated protective layer 32. In particular, the speed control arrangement 200 may be arranged so as to receive the delaminated protective layer 32 from the delamination arrangement 120. For example, the delamination arrangement 120 may be arranged between the support surface 112 of the conveyor device 110 and the speed control arrangement 200 with respect to a transport path of the delaminated protective layer 32.

The speed control arrangement 200 may pull the delaminated protective layer 32 away from the delamination arrangement 120 and/or the support surface 112 by controlling the speed of the delaminated protective layer 32 in a defined manner or with a defined force, thereby assisting in providing the defined delamination force.

In some implementations, the speed control arrangement 200 includes a determination unit having a pair of fixed rolls 210a, 210b, such as a first fixed roll 210a and a second fixed roll 210b, and a moveable roll 212. The first fixed roll 210a, the second fixed roll 210b, and the moveable roll 212 are arranged in, or located on, a triangle and configured to guide the delaminated protective layer 32 coming from the conveyor device 110, in particular the delamination arrangement 120. For example, the delaminated protective layer 32 may first reach the first fixed roll 210a, move to the moveable roll 212, and then move to the second fixed roll 210b. Therefore, due to the triangular arrangement, the first fixed roll 210a, the moveable roll 212, and the second fixed roll 210b may act as divert rolls.

The fixed rolls 210a, 210b are fixed in position, in particular with respect to the delamination arrangement 120 and/or the conveyor device 110. The moveable roll 212 is moveable with respect to the fixed rolls 210a, 210b.

Each of the fixed rolls 210a, 210b and the moveable roll 212 has a respective rotational axis. The rotational axes of all rolls of the fixed rolls 210a, 210b and the moveable roll 212 may be substantially parallel to each other. The term "substantially parallel" relates to a substantially perpendicular orientation of the rotational axes, wherein a deviation of a few degrees, e.g. up to 10° or even up to 15°, from an exact parallel orientation is still considered "substantially parallel". In some embodiments, the rotational axes of all rolls of the fixed rolls 210a, 210b and the moveable roll 212 may be substantially horizontally oriented rotational axes.

In some implementations, the fixed rolls 210a, 210b and the moveable roll 212 may be cylindrical rolls. A cylinder axis of the cylindrical roll may define, or be, the rotational axis of this roll.

The pair of fixed rolls 210a, 210b and the moveable roll 212 are arranged in, or located on, a triangle. This means that each rotational axis corresponds to a respective corner of the triangle.

Preferably, the apparatus 100 is configured to perform a control of the speed of the delaminated protective layer 32 based on a position of the moveable roll 212. For example, if the moveable roll 212 moves towards the pair of fixed rolls 210a, 210b (upward in FIG. 2), it may be determined that the speed control arrangement 200 is pulling or moving the delaminated protective layer 32 to much. In this case, the speed of the delaminated protective layer 32 may be reduced. Likewise, if the moveable roll 212 moves away from the pair of fixed rolls 210a, 210b (downward in FIG. 2), it may be determined that the speed control arrangement 200 is not pulling or moving the delaminated protective layer 32 enough. In this case, the speed of the delaminated protective layer 32 may be increased.

The apparatus 100 may be configured to perform the control of the speed of the delaminated protective layer 32 such that the position of the moveable roll 212 corresponds to a reference position. In some exemplary embodiments, a PID control may be used to control the speed of the delaminated protective layer 32 such that the position of the moveable roll 212 corresponds to the reference position.

Because the moveable roll 212 can move in multiple directions, the moveable roll 212 (or the speed control arrangement 200 as a whole) can be referred to as "dancer".

In some embodiments, the moveable roll 212 is linearly moveable towards a center between the pair of fixed rolls 210, 210b. Alternatively, the moveable roll 212 is circularly moveable, e.g. with respect to a rotational center spaced apart from the moveable roll 212.

In a preferred configuration, the speed control arrangement 200 includes an encoder device 214 configured to determine the position of the moveable roll 212 by detecting a deflection of the moveable roll 212. In some embodiments, the moveable roll 212 is circularly moveable with respect to a rotational center defined by the encoder device 214, as it is illustrated in FIG. 2. In this case, the encoder device 214 may be a rotary encoder. The rotary encoder is an electro-mechanical device that converts the angular position or motion of the moveable roll 212 to an analog or digital output signal used to control the speed of the delaminated protective layer 32.

However, the present disclosure is not limited thereto and the encoder device 214 may be configured to determine the position of a linearly moving moveable roll 212. Even in this case, the encoder device 214 may be a rotary encoder. For example, the rotary encoder may be connected to the moveable roll 212 by means of an elastic element 215 which allows the linear movement of the moveable roll 212 and a rotation of the rotary encoder caused by the linear movement of the moveable roll 212.

According to some embodiments, the speed control arrangement 200 further includes a pair of driven rolls, such as a first driven roll 216a and a second driven roll 216b, configured to guide the delaminated protective layer 32 coming from the determination unit, in particular the second fixed roll 210b. For example, one or more actuators, such as one or more motors, can be provided to drive, i.e. rotate, the pair of driven rolls. In some implementations, the pair of driven rolls may be nip rolls.

The first driven roll 216a and the second driven roll 216b may define a space or gap therebetween through which the delaminated protective layer 32 coming from the determination unit passes. In particular, the delaminated protective layer 32 can be interposed between the first driven roll 216a and the second driven roll 216b such that a mechanical contact between the driven rolls and the delaminated protective layer 32 moves the delaminated protective layer 32 according to a rotation of the first driven roll 216a and the second driven roll 216b. Thereby, the first driven roll 216a and the second driven roll 216b can control the moving speed of the delaminated protective layer 32.

It is therefore understood that, when reference is made to "the moving speed of the delaminated protective layer", this may also refer to the rotational speed of the pair of driven rolls which causes the moving speed of the delaminated protective layer 32.

The rotational axes of the pair of driven rolls 216, 216b may be substantially parallel to the rotational axes of the pair of fixed rolls 210, 210b and the moveable roll 212. For example, the rotational axes of the pair of driven rolls 216, 216b may be substantially horizontally oriented rotational axes.

In some implementations, the first driven roll 216a and the second driven roll 216b may be cylindrical rolls. A cylinder axis of the cylindrical driven roll may define, or be, the rotational axis of this driven roll.

According to some embodiments, one or more guide rolls 150a, 150b can be provided between the delamination arrangement 120 and the speed control arrangement 200 to guide a movement of the delaminated protective layer 32 from the delamination arrangement 120 to the speed control arrangement 200.

According to some embodiments, which can be combined with other embodiments described herein, the apparatus 100 further includes a second lamination arrangement 140 configured for laminating at least one layer onto the article structure 20. In some implementations, the second lamination arrangement 140 may be arranged behind the delamination arrangement 120 with respect to the transport path of the article structure 20. In particular, the first lamination arrangement 130, the delamination arrangement 120 and the second lamination arrangement 140 may be sequentially arranged along the transport path of the article structure 20 and/or along the support surface 112 and/or above the support surface 112.

The second lamination arrangement 140 may include one or more lamination rolls, such as a first lamination roll 140a and a second lamination roll 140b. The first lamination roll 140a and the second lamination roll 140b may define a space or gap therebetween through which at least one layer 40 to be laminated onto the article structure 20 passes. For example, the at least one layer 40 to be laminated onto the article structure 20 may be a release liner. The release liner may be removable to expose e.g. a silicone layer of the wound care article before the wound care article is attached to a body.

Preferably, the first lamination roll 140a and the second lamination roll 140b are arranged above each other in a direction substantially perpendicular to the support surface 112. The term "substantially perpendicular" relates to a substantially perpendicular orientation e.g. of a line on which the first lamination roll 140a and the second lamination roll 140b are arranged, wherein a deviation of a few degrees, e.g. up to 5° or even up to 10°, from an exact perpendicular orientation is still considered as "substantially perpendicular".

Each of the one or more lamination rolls, such as the first lamination roll 140a and the second lamination roll 140b, may have a respective rotational axis. The rotational axes of multiple lamination rolls may be substantially parallel to each other. The term "substantially parallel" relates to a substantially parallel orientation of the rotational axes, wherein a deviation of a few degrees, e.g. up to 10° or even up to 15°, from an exact parallel orientation is still considered "substantially parallel". In some embodiments, the rotational axes of the one or more lamination rolls may be substantially horizontally oriented rotational axes.

In some implementations, the one or more lamination rolls may be cylindrical rolls. A cylinder axis of the cylindrical lamination roll may define, or be, the rotational axis of this lamination roll.

Typically, the rotational axis of the one or more lamination rolls, such as the rotational axes of the first lamination roll 140a and the second lamination roll 140b, are substantially parallel to the support surface 112.

According to the embodiments of the present disclosure, a pressure arrangement (not shown) can be provided e.g. between the first lamination arrangement 130 and the delamination arrangement 120 and/or between the delamination arrangement 120 and the second lamination arrangement 140. The pressure arrangement is explained in detail with respect to FIGs. 4 to 7 and reference is made to this description for details.

According to some embodiments, which can be combined with other embodiments described herein, the apparatus 100 may include at least one separation device (not shown) configured to separate, e.g. cut, the article structure 20 into a plurality of articles. The at least one separation device may be arranged behind the conveyor device 110 with respect to the transport path of the article structure 20. In other embodiments, the at least one separation device is not included in the apparatus 100 and is provided as a separate entity.

According to some embodiments, which can be combined with other embodiments described herein, the articles are wound care articles, such as wound dressings or plasters.

Furthermore, there may be several rows of continuous bands of the article structure which is moved on the support surface of the conveyor. At least the lamination rolls 130a, 130b and lamination rolls 140a, 140b might be used for parallel delaminating or laminated of the several rows of continuous bands, respectively. Also, the delamination rolls 120a, 120b might be used for delaminating the protective layer from the several rows of continuous bands of the article structure. For independently controlling the speed of the delaminated protective layer there might be several speed control arrangements, each controlling the speed of the delaminated protective layer for a single row of the continuous band of the article structure.

FIG. 4A shows a schematic view of an apparatus 400A having a pressure arrangement 410 according to embodiments described herein. The apparatus 400A may be configured similarly to the apparatus shown in FIGs. 2 and 3, and therefore a description of similar or identical aspects will not be repeated.

The first lamination arrangement 130 is configured for laminating at least one layer onto the wound care article structure 20 which is entering the apparatus 400A from the left side in FIG. 4A. The at least one layer may be a single layer. Alternatively, the at least one layer may be a multilayered structure 30, such as the multilayered structure 14 or 30 described with respect to FIGs. 1A and 2.

In the following, the at least one layer is the multilayered structure 30. However, the following description also applies to a single layer which is laminated onto the wound care article structure 20. This single layer or multilayered structure 30 might be called wound cover layer.

In some embodiments, the multilayered structure 30 may include the multilayered structure 14 shown in FIG. 1A and the protective layers 31, 32 on its sides. The first lamination arrangement 130 may be configured to remove the first protective layer 31 from the multilayered structure 30 which faces the wound care article structure 20 before the multilayered structure is laminated onto the wound care article structure 20.

After the multilayered structure 30 has been laminated onto the wound care article structure 20, the modified wound care article structure 20 having the multilayered structure thereon reaches the pressure arrangement 410. The pressure arrangement 410 is used to strengthen a connection between the multilayered structure and the underlying part of the wound care article structure 20, such as a carrier/backsheet and/or an adhesive layer and/or a wound pad, by applying pressure to the layer arrangement. This can ensure that the multilayered structure 30 does not detach from the wound care article structure 20 in a subsequent process step in which e.g. the protective layer 32 is removed or delaminated. In particular, due to the pressurization, an adhesion force between the multilayered structure 30 and the wound care article structure 20 can be greater than a delamination force so that the multilayered structure does not detach from the wound care article structure 20 due to the application of the delamination force. Furthermore, due to the pressure arrangement 410 an inclusion of air between the multilayered structure 30 and the wound care article structure 20 is prevented or at least reduced.

The pressure arrangement 410 includes one or more pressure rolls configured to apply a pressure to the multilayered structure to fix the multilayered structure to the wound care article structure 20. For example, the pressure arrangement 410 is configured to apply the pressure to the multilayered structure to press an acrylic layer of the multilayered structure (e.g. the first layer 15 in FIG. 1A) against an acrylic layer of the wound care article structure 20 (e.g. the intermediate layer 12 in FIG. 1A).

The support surface 112 includes a first side configured for supporting the wound care article structure 20, in particular a non-woven carrier of the wound care article structure 20. The support surface 112 further includes a second side opposite the first side. At least one first pressure roll 412 of the one or more pressure rolls is located at the first side of the support surface 112. The at least one first pressure roll 412 may be configured to directly contact the multilayered structure press the multilayered structure against the underlaying layer(s) or part(s) of the wound care article structure 20.

The multilayered structure may still have the second protective layer 32 thereon so that the at least one first pressure roll 412 contacts the second protective layer 32. The second protective layer 32 can be made of a material which does not adhere to the at least one first pressure roll 412. For example, the second protective layer 32 may be a siliconized paper. This can improve the pressurization process and prevent a detachment of the uppermost layer of the multilayered structure due to an adherence to the at least one first pressure roll 412.

The wound care article structure 20 may have a plurality of wound pads 13 arranged in series along the wound care article structure 20, e.g. the x-direction (direction 2) in FIG. 4A. The plurality of wound pads 13 are spaced apart from each other in the x-direction. It should thus be understood that the at least one first pressure roll 412 may not only pressurize an area of the wound pad and/or an area surrounding the wound pad (e.g. lateral sides of the wound pads), but also an area between the plurality of wound pads 13.

The wound pads 13 may have a predetermined height which protrudes from the carrier. Depending on the application the height might differ. The wound pads may have a 3D- structure and/ may have an irregular surface. Thus, by using the one or more pressure roll sufficient pressure is applied on different lamination levels to ensure a reliable contact between the multilayered structure 30 and the wound care article structure 20, in particular between the wound pad 13 and the multilayered structure 30.

By using wound pads having a large thickness and different height levels different pressure rolls could be used only for pressing the wound pad against the wound care article structure. Thus, different and adapted pressure levels on one or more lamination levels can be provided to the complex wound pad to ensure a reliable adhesion of the wound cover layer (multilayered structure 30) onto the wound pads 13. This, is in particular necessary if the elastic cover material of the pressure rolls could not adapt to the height profile of the wound pad, i.e. if the height profile of the wound pads 13 is larger than the elasticity of the cover material of the pressure rolls.

In some implementations, the one or more pressure rolls are a plurality of pressure rolls, such as one or more pairs of pressure rolls. At least one second pressure roll 414 of the plurality of pressure rolls may be located at the second side of the support surface 112. The support surface 112 and the wound care article structure 20 may be interposed between the at least one first pressure roll 412 and the at least one second pressure roll 414 to apply the pressure from above and below the wound care article structure 20.

Preferably, a respective first pressure roll 412 and a respective second pressure roll 414 constitute a pair of pressure rolls and are arranged above each other in a direction substantially perpendicular to the support surface 112. The term "substantially perpendicular" relates to a substantially perpendicular orientation e.g. of a line on which the pressure rolls of the pair of pressure rolls are arranged, wherein a deviation of a few degrees, e.g. up to 5° or even up to 10°, from an exact perpendicular orientation is still considered as "substantially perpendicular".

In the example of FIG. 4A, one single pair of pressure rolls is provided.

Each of the one or more pressure rolls, such as the at least one first pressure roll 412 and the at least one second pressure roll 414, may have a respective rotational axis. The rotational axes of multiple pressure rolls may be substantially parallel to each other. The term "substantially parallel" relates to a substantially parallel orientation of the rotational axes, wherein a deviation of a few degrees, e.g. up to 10° or even up to 15°, from an exact parallel orientation is still considered "substantially parallel". In some embodiments, the rotational axes of the one or more pressure rolls may be substantially horizontally oriented rotational axes.

In some implementations, the one or more pressure rolls may be cylindrical rolls. A cylinder axis of the cylindrical pressure roll may define, or be, the rotational axis of this particular pressure roll.

Typically, the rotational axes of the one or more pressure rolls, such as the rotational axes of the at least one first pressure roll 412 and the rotational axes of the at least one second pressure roll 414, are substantially parallel to the support surface 112.

The arrangement of first pressure rolls and second pressure rolls may be used in a case in which the support surface 112 is a flexible support surface. For example, the flexible support surface may be provided by a belt 116, such as a rubber belt.

In the example of FIG. 4A, the pressure arrangement 410 is located between the first lamination arrangement 130 and the delamination arrangement 120. However, the present disclosure is not limited thereto and the pressure arrangement 410 may be located between the delamination arrangement 120 and the second lamination arrangement 140 and/or behind the second lamination arrangement 140 in a transport direction of the wound care article structure 20.

According to some embodiments, which can be combined with other embodiments described herein, the apparatus can include one pressure arrangement or can include two or more pressure arrangements. The two or more pressure arrangements can be arranged at suitable locations. For example, one pressure arrangement can be arranged between the first lamination arrangement 130 and the delamination arrangement 120 and/or another pressure arrangement can be arranged between the delamination arrangement 120 and the second lamination arrangement 140 and/or yet another pressure arrangement can be arranged behind the second lamination arrangement 140 in a transport direction of the wound care article structure 20. Other locations are also possible.

FIG. 4B shows a schematic view of an apparatus 400B having a pressure arrangement 420 according to further embodiments described herein. The apparatus 400B of FIG. 4B is similar to the apparatus 400A of FIG. 4A, and therefore a description of similar or identical aspects is not repeated.

In the example of FIG. 4A, one single pair of pressure rolls is provided. However, as is shown in FIG.4B, the pressure arrangement 420 may include two or more pairs of pressure rolls, such as a first pair having a first pressure roll 412 and a second pressure roll 414 and a second pair having another first pressure roll 422 and another second pressure roll 424.

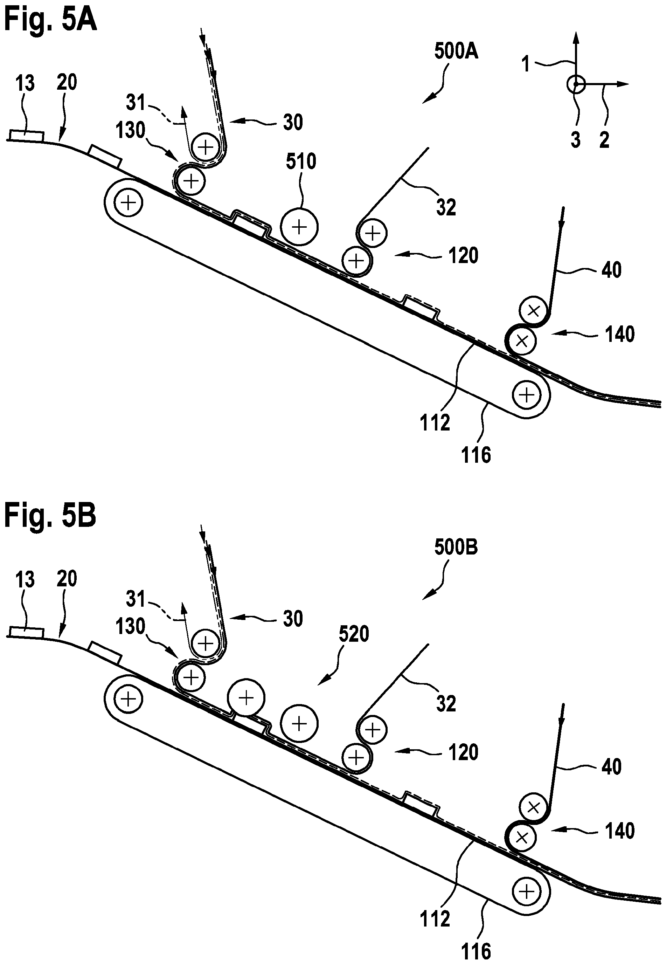

The two or more pairs of pressure rolls can be sequentially arranged along the transport path of the wound care article structure 20, e.g. in x direction (2 in Fig. 5a).

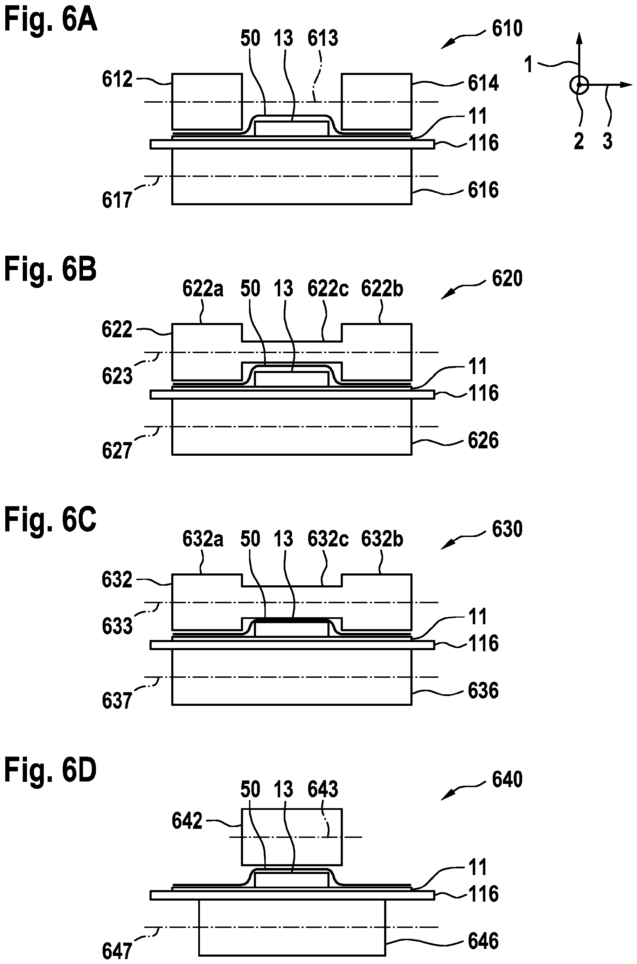

The two or more pairs of pressure rolls can be arranged to pressurize different portions or areas of the at least one layer, such as the multilayered structure 30, against the underlying part of the wound care article structure 20. For example, one pair of pressure rolls can be configured to pressurize portions adjacent to the wound pads 13 with respect to a width direction of the wound care article structure 20. The width direction of the conveyor can be a direction perpendicular to the transport direction of the wound care article structure 20 (z-direction (3)). Accordingly, this pair of pressure rolls may exclude the wound pads 13 from pressurization, i.e., the pair of pressure rolls does not pressurize the wound pads 13. Thereby, an area of the multilayered structure surrounding the wound pads 13 on a left side and a right side thereof can be pressed against the wound care article structure 20 to adhere the multilayered structure to the wound care article structure 20. An exemplary roll configuration for this purpose is shown in FIGs. 6A and 6B.

Another pair of pressure rolls can be configured to pressurize a portion or an area above the wound pads 13 and optionally between the wound pads 13. Thereby, the multilayered structure above the wound pads 13 can be pressed against the wound pads 13 to adhere the multilayered structure to the wound pads 13. An exemplary roll configuration for this purpose is shown in FIG. 6D.

According to some embodiments, which can be combined with other embodiments described herein, the apparatus can include another station configured for a longitudinal edge sealing, i.e., a sealing of an area of the multilayered structure surrounding the wound pads on the left side and the right side thereof. This can provide an increased process reliability and/or reduce wear of the pressure arrangement.

FIG. 5A shows a schematic view of an apparatus 500A having a pressure arrangement 510 according to further embodiments described herein. The apparatus 500A is similar to the apparatus 400A of FIG. 4A and a description of similar or identical aspects is therefore not repeated.

In some implementations, the support surface 112 can be a substantially inflexible support surface. For example, the support surface 112 can be a metal surface. In this case, the one or more pressure rolls may be located only at the first side of the support surface 112. In other words, the at least one second pressure roll described with respect to FIGs. 4A and 4B can be omitted because the substantially inflexible support surface provides sufficient support such that the at least one first pressure roll of the pressure arrangement 510 can press the multilayered structure against the wound care article structure 20.

FIG. 5B shows a schematic view of an apparatus 500B having a pressure arrangement 520 according to further embodiments described herein. The apparatus 500B is similar to the apparatuses 400B and 500A of FIGs. 4B and 5A, and a description of similar or identical aspects is therefore not repeated.

The pressure arrangement 520 may include two or more first pressure rolls. The two or more first pressure rolls can be sequentially arranged along the transport path of the wound care article structure 20.

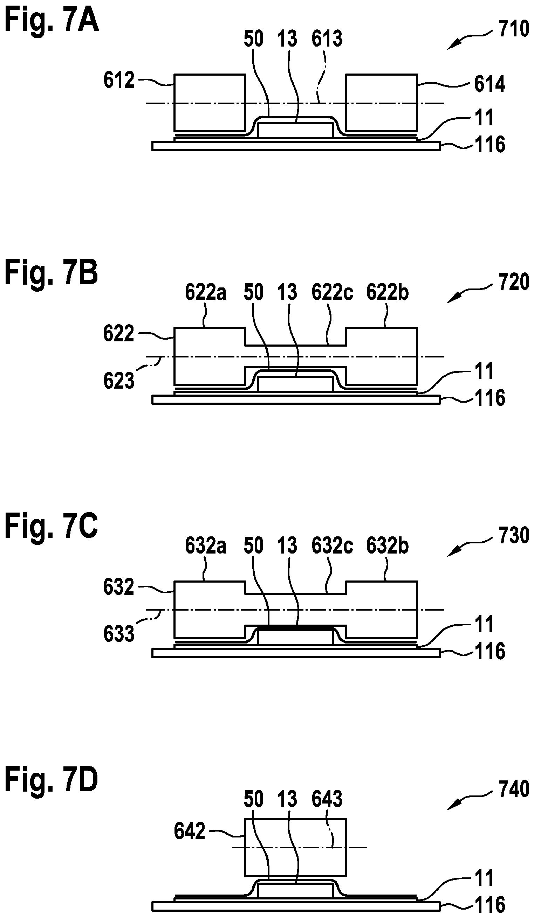

The two or more first pressure rolls can be arranged to pressurize different portions or areas of the at least one layer, such as the multilayered structure 30, against the underlying part of the wound care article structure 20. For example, one first pressure roll can be configured to pressurize portions adjacent to the wound pads 30 with respect to a width direction of the wound care article structure 20. The width direction can be a direction perpendicular to the transport direction of the wound care article structure 20 (z-direction). Accordingly, this first pressure roll may exclude the wound pads 13 from pressurization, i.e., this first pressure roll does not pressurize the wound pads 13. Thereby, an area of the multilayered structure surrounding the wound pads 13 on a left side and a right side thereof can be pressed against the wound care article structure 20 to adhere the multilayered structure to the wound care article structure 20. An exemplary roll configuration for this purpose is shown in FIGs. 7A and 7B.

Another first pressure roll can be configured to pressurize a portion or an area above the wound pads 13 and optionally between the wound pads 13. Thereby, the multilayered structure above the wound pads 13 can be pressed against the wound pads 13 to adhere the multilayered structure to the wound pads 13. An exemplary roll configuration for this purpose is shown in FIG. 7D.

FIGs. 6A-D show various pressure rolls of pressure arrangements according to embodiments described herein. FIGs. 6A-D show front views of the pressure arrangements compared to the side views of FIGs. 2, 4 and 5. The pressure arrangements of FIGs. 6A-D may be implemented in the apparatuses of FIGs. 4A and 4B. The pressure rolls as shown in Fig. 6A or 6D have different diameter or one pressure roll as shown in Fig. 6B or 6C have different diameters on the same roll. Thus, different lamination levels are realized to apply a specific pressure to specific portions of the multilayered structure 30.

For clarity, in FIGs. 6A-D, gaps are shown between some of the rolls and some of the layers. It should be clear that these gaps are not present in areas where the layers are pressed against each other. In other words, despite the gaps shown in FIGs. 6A-D are shown, there is direct contact between the roll(s) and the layer(s) in some areas.

In the following, the at least one layer 50 is shown. The at least one layer 50 may be a single layer or a multilayered structure, such as the multilayered structure 30 without the first protective layer 31 and optionally without the second protective layer 32.

The support surface 112 may be a flexible support surface as it is described with respect to FIGs. 4A and 4B. For example, the support surface 112 can be provided by a flexible belt 116 of a belt conveyor. Therefore, at least one first pressure roll and at least one second pressure roll are provided on opposite sides of the flexible belt 116 in order to be able to pressurize the wound care article structure and the at least one layer 50.

The wound care article structure and the at least one layer 50 (e.g., the multilayered structure of FIGs. 2-5) laminated thereon are interposed between the at least one first pressure roll and the at least one second pressure roll such that they are pressed against each other. For example, the at least one layer 50 can be pressed against the carrier 11 or an adhesive layer thereon. Both the at least one layer 50 and the adhesive layer on the carrier 11 may be acrylic layers. The pressure arrangement can press both layers against each other so that an adherence between the layers is enhanced.

FIG. 6A shows a pressure arrangement 610 having two first pressure rolls 612, 614 located at the first side of the support surface and a second pressure roll 616 located at the second side of the support surface. Each of the two first pressure rolls 612, 614 and the second pressure roll 616 has a constant diameter. The diameter of each pressure roll may be defined in a plane perpendicular to a rotational axis of the respective roll.

The two first pressure rolls 612, 614 are rotatable around the same rotational axis 613. The two first pressure rolls 612, 614 are spaced apart from each other in a direction along the rotational axis 613, e.g. the horizontal direction 3 perpendicular to a transport direction of the wound care article structure. In this example, the two first pressure rolls 612, 614 are separate entities or elements.

The wound pads 13 may pass through a space between the two first pressure rolls 612, 614. Accordingly, the two first pressure rolls 612, 614 may exclude the wound pads 13 from pressurization. To achieve this, a distance between the two first pressure rolls 612, 614 in the direction along the rotational axis 613 can be larger than a width of the wound pad(s) 13 in the direction along the rotational axis 613.

The second pressure roll 616 may extend below the two first pressure rolls 612, 614 and the space through which wound pads 30 pass. Thus, the second pressure roll 616 serves as a back pressure roll.

The first pressure rolls 612, 614 may be metal-coated rolls. For example, a substrate material, such as a synthetic or plastic material, can be covered with a metal layer. Preferably, the metal may be aluminum. Additionally, the second pressure roll 616 may be made of a silicone material. In some examples, the second pressure roll 616 may be a smooth silicone roll. The silicone material may have a hardness of 75-80 Shore.

Alternatively, the first pressure rolls 612, 614 and the second pressure roll 616 may all be made of a silicone material. In some examples, the first pressure rolls 612, 614 and the second pressure roll 616 may be smooth silicone rolls. The silicone material may have a hardness of 75-80 Shore.

FIG. 6B shows a pressure arrangement 620 having a (one) first pressure roll 622 located at the first side of the support surface and a second pressure roll 626 located at the second side of the support surface.

The second pressure roll 626 has a constant diameter and is rotatable around a rotational axis 627. The second pressure roll 616 may extend below the first pressure roll 622 and the space through which wound pads 30 pass. Thus, the second pressure roll 626 serves as a back pressure roll.

The first pressure roll 622 has a variable diameter. In particular, the first pressure roll 622 has two protruding portions 622a, 622b and a recessed portion 622c between the two protruding portions 622a, 622b. A diameter of each of the protruding portions 622a, 622b is larger than a diameter of the recessed portion 622c.

The recessed portion 622c may provide an accommodation space for the wound pads 13. In particular, a width of the recessed portion 622c in a direction substantially parallel to the rotational axis 623 of the first pressure roll 622 may be equal to or larger than a width of the wound pads 13 in a direction substantially parallel to the rotational axis 623. Accordingly, the two protruding portions 622a, 622b can pressurize the left side and the right side of the wound pads 13 with respect to the transport direction of the wound care article structure.

In the example of FIG. 6B the recessed portion 622c may be configured such that the first pressure roll 622 neither contacts nor pressurizes a top surface of the wound pads 13 passing through the recessed portion 622c. Further, the recessed portion 622c may neither contact nor pressurize an area between the wound pads 13 in the transport direction. To achieve this, a depth of the recessed portion 622c in a direction substantially perpendicular to the rotational axis 627 of the first pressure roll 622 may be equal to, or larger than, a height of the wound pad 13 in the direction substantially perpendicular to the rotational axis 627. Accordingly, the first pressure roll 622 may have a geometry that excludes the wound pads 13 from pressurization.

In the example of FIG. 6B a transition from the protruding portions 622a, 622b to the recessed portion 622c is formed as a step. However, the present disclosure is not limited thereto and the transition from the protruding portions 622a, 622b to the recessed portion 622c can be sloped, curved or round.

The first pressure roll 622 may be a metal-coated roll. For example, a substrate material, such as a synthetic or plastic material, can be covered with a metal layer. Preferably, the metal may be aluminum. Additionally, the second pressure roll 626 may be made of a silicone material. In some examples, the second pressure roll 626 may be a smooth silicone roll. The silicone material may have a hardness of 75-80 Shore.

Alternatively, the first pressure roll 622 and the second pressure roll 626 may all be made of a silicone material. In some examples, the first pressure roll 622 and the second pressure roll 626 may be smooth silicone rolls. The silicone material may have a hardness of 75-80 Shore.

FIG. 6C shows a pressure arrangement 630 which is similar to the pressure arrangement of FIG. 6B and therefore a description of identical or similar aspects is not repeated.

The pressure arrangement 630 includes a first pressure roll 632 rotatable around a rotational axis 633 and a second pressure roll 636 rotatable around a rotational axis 637.

In the example of FIG. 6C, a depth of the recessed portion 632c of the first pressure roll 632 in a direction substantially perpendicular to the rotational axis 633 of the first pressure roll 632 is smaller than a height of the wound pad 13 in the direction substantially perpendicular to the rotational axis 633 of the first pressure roll 632. Accordingly, the protruding portions 632a, 632b of the first pressure roll 632 may pressurize the top surface of the wound pad 13 to better adhere the at least one layer 50 to the wound pads 13. This configuration allows to pressure all areas in a single step using a single pressure roll.

In the example of FIG. 6B a transition from the protruding portions 632a, 632b to the recessed portion 632c is formed as a step. However, the present disclosure is not limited thereto and the transition from the protruding portions 632a, 632b to the recessed portion 632c can be sloped, curved or round.

The first pressure roll 632 may be a metal-coated roll. For example, a substrate material, such as a synthetic or plastic material, can be covered with a metal layer. Preferably, the metal may be aluminum. Additionally, the second pressure roll 636 may be made of a silicone material. In some examples, the second pressure roll 636 may be a smooth silicone roll. The silicone material may have a hardness of 75-80 Shore.

Alternatively, the first pressure roll 632 and the second pressure roll 636 may all be made of a silicone material. In some examples, the first pressure roll 632 and the second pressure roll 636 may be smooth silicone rolls. The silicone material may have a hardness of 75-80 Shore.

FIG. 6D shows a pressure arrangement 640 which includes a first pressure roll 642 rotatable around a rotational axis 643 and a second pressure roll 646 rotatable around a rotational axis 647. The first pressure roll 642 has a constant diameter. The first pressure roll 642 may provide the function of the recessed portion described with respect to FIG. 6C. In particular, the first pressure roll 642 may pressurize the top surface of the wound pad 13 to better adhere the at least one layer 50 to the wound pad 13. Optionally, the first pressure roll 642 may pressurize the area between the wound pads 13 in the transport direction.

According to some embodiments, the pressure arrangement 610 of FIG. 6A and the pressure arrangement 640 of FIG. 6D may be sequentially arranged along the transport path of the wound care article structure. The pressure arrangement 610 of FIG. 6A may pressurize the portions of the wound care article structure adjacent to and/or surrounding the wound pad(s) 13, and the pressure arrangement 640 of FIG. 6D may pressurize the top surface of the wound pad(s) 13 and the area between the wound pads 13.

The first pressure roll 642 may be a metal-coated roll. For example, a substrate material, such as a synthetic or plastic material, can be covered with a metal layer. Preferably, the metal may be aluminum. The present disclosure is not limited to a metal coating and other suitable coating can be used. Additionally, the second pressure roll 646 may be made of a silicone material. In some examples, the second pressure roll 646 may be a smooth silicone roll. The silicone material may have a hardness of 75-80 Shore.

Alternatively, the first pressure roll 642 and the second pressure roll 646 may all be made of a silicone material. In some examples, the first pressure roll 642 and the second pressure roll 646 may be smooth silicone rolls. The silicone material may have a hardness of 75-80 Shore.

FIGs. 7A-D show various pressure rolls of pressure arrangements according to further embodiments described herein. FIGs. 7A-D show front views of the pressure arrangements compared to the side views of FIGs. 2, 4 and 5.

The embodiments of FIGs. 7A-D are similar to the embodiments of FIGs. 6A-D, respectively, and a description of similar or identical features is omitted. In the example of FIGs. 7A-D the support surface is an inflexible support surface, such as a metal surface. Therefore, the second pressure rolls of the embodiments of FIGs. 6A-D are omitted. The remaining features are essentially the same.

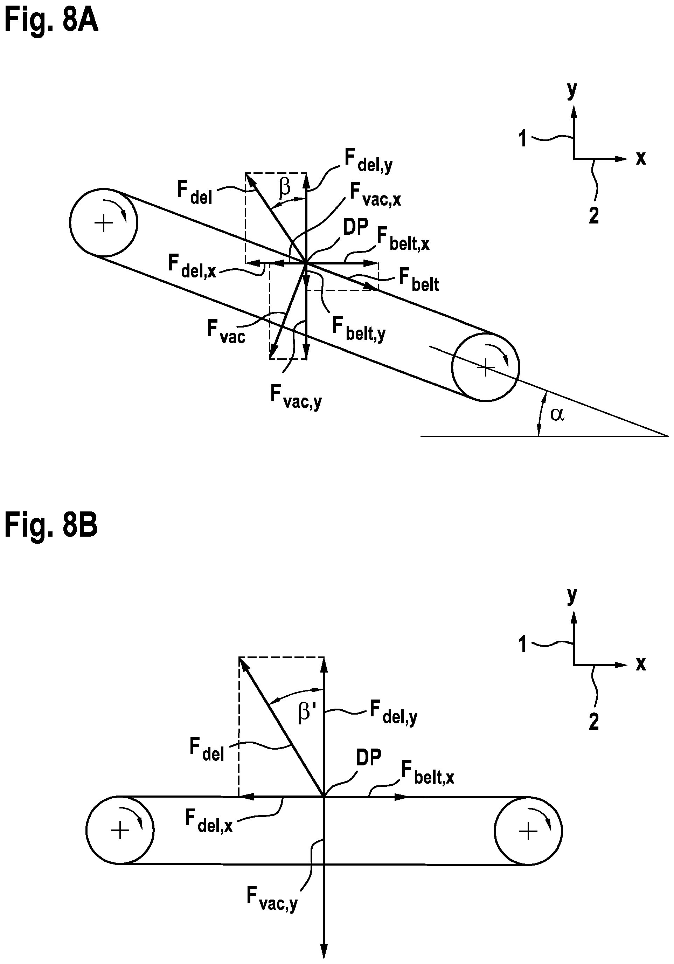

FIG. 8A shows forces in an apparatus for removing at least one layer from an article structure according to embodiments described herein. The force amplitudes and force ratios may be exaggerated or understated for the purpose of clarity of the figures and are therefore not a scale representation of the force amplitudes and force ratios.

In the example of FIG. 8A, the support surface is inclined with respect to the horizontal direction by an inclination angle $\alpha$.

Generally, the term "vertical direction" or "vertical orientation" is understood to distinguish over "horizontal direction" or "horizontal orientation". That is, the vertical direction 1 or vertical orientation relates to a vertical orientation of the forces or force components. The horizontal direction 2 is perpendicular to the vertical direction 1. The vertical direction 1 is parallel to the force of gravity.

The following forces may act on a given point (i.e., the delamination point DP) of the article structure:

- delamination force $F_{del}$: exerted by the delamination arrangement;
- belt force $F_{belt}$: exerted by the support surface (belt) and its movement; and
- holding force $F_{vac}$: exerted by the suction mechanism configured to hold the article structure at the support surface.

These forces act on the delamination point DP, at which the protective layer detaches from the article structure.

Each force can be presented by a vector, wherein each vector is composed of a horizontal component (index "x") and a vertical component (index "y"). The vectors and their respective components are shown in FIG. 8A.

In detail, the delamination force $F_{del}$ has a first delamination force component $F_{del,y}$ in the vertical direction 1 and a second delamination force component $F_{del,x}$ in the horizontal direction 2. The belt force $F_{belt}$ has a vertical force component $F_{belt,y}$ and a horizontal force component $F_{belt,x}$. The holding force (e.g. suction force) $F_{vac}$ has a vertical force component $F_{vac,y}$ and a horizontal force component $F_{vac,x}$.

The sum of the horizontal force components acting on the article structure at the delamination point DP is thus given as:

$$\sum F_x = F_{belt,x} - F_{vac,x} - F_{del,x} \qquad (1)$$

The sum of the vertical force components acting on the article structure at the delamination point DP is given as:

$$\sum F_y = F_{del,y} - F_{belt,y} - F_{vac,y} \qquad (2)$$

According to the embodiments of the present disclosure, the first delamination force component $F_{del,y}$ is smaller than a vertical force component generated by the conveyor device (in the present example the sum of $F_{belt,y}$ and $F_{vac,y}$) and acting on the article structure 2 in a direction opposite to the first delamination force component $F_{del,y}$.

In the present example, this condition can be expressed as follows:

$$|F_{del,y}| < |F_{belt,y}| + |F_{vac,y}| \qquad (3)$$

Such an adjustment of the delamination force to satisfy the above conditions ensures that the article structure adheres to the support surface of the conveyor and does not lift off. Accordingly, the production speed and thus the yield of a manufacturing system can be increased.

The adjustment of the delamination force may be achieved by adjusting a delamination angle β defined between a direction or vector of the delamination force $F_{del}$ and its first delamination force component (vector component) $F_{del,y}$. In some embodiments, the delamination angle β may be 10° or higher and/or less than 90°. For example, the delamination angle is 15° or higher, 20° or higher, 25° or higher, 30° or higher, 35° or higher, 40° or higher, or 45° or higher. In any case, the delamination angle may be less than 90°.

In order to adjust the delamination force and/or the delamination angle β, the inclination angle α of the support surface with respect to the horizontal direction 1 may be adjusted. In some embodiments, in order to satisfy the above condition (3), the inclination angle may be 45° or less and more than 0°. For example, the inclination angle may be 40° or less, 35° or less, 30° or less, 25° or less, 20° or less, 15° or less, or 10° or less. In any case, the inclination angle α may be greater than 0°. In a particular embodiment, the inclination angle α may be about 17°.

FIG. 8B shows forces in an apparatus for removing at least one layer from an article structure according to further embodiments described herein. In the example of FIG. 8B, the support surface is horizontally oriented, i.e., not inclined. Thus, the holding force $F_{vac}$ has only a vertical component $F_{vac,y}$, and the belt force $F_{belt}$ only has a horizontal component $F_{del,x}$.

In this case, the delamination angle β may be adjusted by adjusting a direction of the vector of the delamination force $F_{del}$. The direction of the vector of the delamination force $F_{del}$ can be adjusted by a geometrical configuration and/or arrangement of the delamination arrangement with respect to the support surface of the conveyor device.

In view of the above, the embodiments of the present disclosure may adjust a delamination force used to peel a temporary protective layer from the article structure. In particular, the delamination force is adjusted so that the article structure adheres to the support surface of the conveyor and does not lift off. Accordingly, the production speed and thus the yield of a manufacturing system can be increased.

Furthermore, the embodiments of the present disclosure may press a layer or multilayered structure that has been laminated onto a wound care article structure against said wound care article structure. Thereby, an adhesion force between the laminated layer or multilayered structure and the wound care article structure can be enhanced so that a subsequent removal of a protective layer does not result in a detachment of the laminated layer or multilayered structure from the wound care article structure. Accordingly, the production speed and thus the yield of a manufacturing system can be increased.

While the foregoing is directed to embodiments of the disclosure, other and further embodiments of the disclosure may be devised without departing from the basic scope thereof, and the scope thereof is determined by the claims that follow.

**Claims**

1. Apparatus (100, 400A, 400B, 500A, 500B) for laminating at least one layer (30, 50) onto a wound care article structure (20), comprising:

   a conveyor device (110) having a support surface (112) configured for supporting the wound care article structure (20);
   a first lamination arrangement (130) configured for laminating at least one layer (30, 50) having at least one protection layer (32) onto the wound care article structure (20);
   a pressure arrangement (410, 420, 510, 520, 610, 620, 630, 640, 710, 720, 730, 740) having one or more pressure rolls configured to apply a pressure to the at least one layer (30, 50) laminated onto the wound care article structure (20) to fix the at least one layer (30, 50) to the wound care article structure (20), wherein the one or more pressure rolls are adjustable in their distance to the wound care article structure (20) or in their distance to a carrier (11) of the wound care article structure (20); and

a delamination arrangement (120) configured for delaminating the protective layer (32) off the wound care article structure (20) having the at least one layer (30, 50) laminated thereon.

2. The apparatus (100, 400A, 400B, 500A, 500B) of claim 1, wherein the at least one layer (30, 50) is a multilayered structure (30), the pressure arrangement (410, 420, 510, 520, 610, 620, 630, 640, 710, 720, 730, 740) is configured to apply the pressure to the multilayered structure (30) to press a first layer (15) of the multilayered structure (30) against the wound care article structure (20).

3. The apparatus (100, 400A, 400B, 500A, 500B) of claim 2, wherein the first and second layers (12, 15) are pressure sensitive adhesive layers.

4. The apparatus (100, 400A, 400B, 500A, 500B) of any one of the preceding claims, wherein the support surface (112) includes a first side configured for supporting the wound care article structure (20) and a second side opposite the first side, and wherein at least one first pressure roll (412, 422, 612, 614, 622, 632, 642) of the one or more pressure rolls is located at the first side of the support surface (112).

5. The apparatus (100, 400A, 400B) of claim 4, wherein the one or more pressure rolls are a plurality of pressure rolls, and at least one second pressure roll (414, 424, 616, 626, 636, 646) of the plurality of pressure rolls is located at the second side of the support surface (112), and wherein the support surface (112) and the wound care article structure (20) are interposed between the at least one first pressure roll (412, 422, 612, 614, 622, 632, 642) and the at least one second pressure roll (414, 424, 616, 626, 636, 646).

6. The apparatus (100, 400A, 400B) of claim 4 or 5, wherein the support surface (112) is a flexible support surface; or the support surface (112) is a substantially inflexible support surface; and/or the one or more pressure rolls are located only at the first side of the support surface (112).

7. The apparatus (100, 400A, 400B) of any one of the preceding claims, wherein one or more pressure rolls of the pressure arrangement (410, 420, 510, 520, 610, 620, 630, 640, 710, 720, 730, 740) provide pressure to the at least one layer (30, 50) at different lamination levels.

8. The apparatus (100, 400A, 400B, 500A, 500B) of any one of claims 4 to 7, wherein the at least one first pressure roll includes at least one constant diameter roll (612, 614, 642) having a substantially constant diameter and/or the at least one first pressure roll includes at least one variable diameter roll (622, 632) having different diameters.

9. The apparatus (100, 400A, 400B, 500A, 500B) of claim 8, wherein the at least one variable diameter roll (622, 632) has at least one protruding portion (622a, 622b, 632a, 632b) and at least one recessed portion (622c, 632c), wherein a diameter of the at least one protruding portion (622a, 622b, 632a, 632b) is larger than a diameter of the at least one recessed portion (622c, 632c).

10. The apparatus (100, 400A, 400B, 500A, 500B) of claim 9, wherein:

a width of the at least one recessed portion (622c, 632c) in a direction substantially parallel to a rotational axis (623, 633) of the at least one variable diameter roll (622, 632) is equal to or larger than a width of an elevated pad portion (13) of the wound care article structure (20); and/or

a depth of the at least one recessed portion (622c, 632c) in a direction substantially perpendicular to the rotational axis (623, 633) of the at least one variable diameter roll (622, 632) is less, equal to, or larger than a height of the elevated pad portion (13) of the wound care article structure (20); and/or

an area of the at least one recessed portion (622c, 632c) is adapted to the form of the elevated pad portion (13) of the wound care article structure (20).

11. The apparatus (100, 400A, 400B, 500A, 500B) of any one of claims 7 to 9, wherein the pressure arrangement includes the at least one variable diameter roll (622) and the at least one constant roll (642) arranged in series along a transport path of the wound care article structure (20).

12. The apparatus (100, 400A, 400B, 500A, 500B) of any one of the preceding claims,

wherein the delamination arrangement (120) includes one or more delamination rolls (120a, 120b) or one or more delamination blades; and/or

wherein the delamination arrangement (120) is configured for delaminating the protective layer (32) off the wound care article structure (20) by applying a delamination force ($F_{del}$) to the protective layer (32),

wherein the delamination force ($F_{del}$) has a first delamination force component ($F_{del,y}$) in a vertical direction (1) and a second delamination force component ($F_{del,x}$) in a horizontal direction (2), and the first delamination force component ($F_{del,y}$) is smaller than a vertical force component ($F_{belt,y}$; $F_{vac,y}$) generated by the conveyor device (110) and acting on the wound care article structure (20) in a direction opposite to the first delamination force component ($F_{del,y}$).

13. The apparatus (100, 400A, 400B, 500A, 500B) of claim 12, further including at least one of:

    a second lamination arrangement (140) configured for laminating at least one further layer (40) onto the wound care article structure (20) after the protective layer (32) has been delaminated off the wound care article structure (20) by means of the delamination arrangement (120); and/or
    at least one separation device configured to separate the wound care article structure (20) into a plurality of wound care articles.

14. Method for laminating at least one layer (30, 50) onto a wound care article structure (20), comprising:

    laminating at least one layer (30, 50) onto a wound care article structure (20) supported on a support surface (112) of a conveyor device (110);
    applying a pressure to the at least one layer (30, 50) using one or more pressure rolls to fix the at least one layer (30, 50) having at least one protective layer (32) to the wound care article structure (20) while the wound care article structure (20) is supported on the support surface (112) of the conveyor device (110), wherein the one or more pressure rolls are adjustable in their distance to the wound care article structure (20) or in their distance to a carrier (11) of the wound care article structure (20); and
    delaminating the protective layer (32) off the wound care article structure (20) having the at least one layer (30, 50) laminated thereon.

**Patentansprüche**

1. Vorrichtung (100, 400A, 400B, 500A, 500B) zum Laminieren wenigstens einer Lage (30, 50) auf eine gewickelte Pflegeartikelstruktur (20), wobei die Vorrichtung Folgendes umfasst:

    eine Transportvorrichtung (110), die eine tragende Fläche (112) aufweist, die konfiguriert ist, die gewickelte Pflegeartikelstruktur (20) zu tragen;
    eine erste Laminieranordnung (130), die konfiguriert ist, wenigstens eine Lage (30, 50), die wenigstens eine Schutzlage (32) hat, auf die gewickelte Pflegeartikelstruktur (20) zu laminieren;
eine Druckanordnung (410, 420, 510, 520, 610, 620, 630, 640, 710, 720, 730, 740), die eine oder mehrere Andruckwalzen hat, die konfiguriert sind, auf die wenigstens eine Lage (30, 50), die auf die gewickelte Pflegeartikelstruktur (20) laminiert worden ist, Druck auszuüben, um die wenigstens eine Lage (30, 50) an der gewickelten Pflegeartikelstruktur (20) zu befestigen, wobei der Abstand der einen oder der mehreren Andruckwalzen zu der gewickelten Pflegeartikelstruktur (20) oder zu einem Träger (11) der gewickelten Pflegeartikelstruktur (20) eingestellt werden kann; und
eine Delaminieranordnung (120), die konfiguriert ist, die Schutzlage (32) von der gewickelten Pflegeartikelstruktur (20), die die wenigstens eine Lage (30, 50) aufweist, die darauf laminiert worden ist, zu delaminieren.

2. Vorrichtung (100, 400A, 400B, 500A, 500B) nach Anspruch 1, wobei die wenigstens eine Lage (30, 50) eine mehrlagige Struktur (30) ist, wobei die Druckanordnung (410, 420, 510, 520, 610, 620, 630, 640, 710, 720, 730, 740) konfiguriert ist, den Druck auf die mehrlagige Struktur (30) auszuüben, um eine erste Lage (15) der mehrlagigen Struktur (30) an die gewickelte Pflegeartikelstruktur (20) zu pressen.

3. Vorrichtung (100, 400A, 400B, 500A, 500B) nach Anspruch 2, wobei die erste und die zweite Lage (12, 15) druckempfindliche Klebstofflagen sind.

4. Vorrichtung (100, 400A, 400B, 500A, 500B) nach einem der vorhergehenden Ansprüche, wobei die tragende Fläche (112) eine erste Seite, die konfiguriert ist, die gewickelte Pflegeartikelstruktur (20) zu tragen, und eine zweite Seite gegenüber der ersten Seite umfasst und wobei wenigstens eine erste Andruckwalze (412, 422, 612, 614, 622, 632, 642) der einen oder mehreren Andruckwalzen auf der ersten Seite der tragenden Fläche (112) angeordnet ist.

5. Vorrichtung (100, 400A, 400B) nach Anspruch 4, wobei die eine oder die mehreren Andruckwalzen mehrere Andruckwalzen sind, wobei wenigstens eine zweite Andruckwalze (414, 424, 616, 626, 636, 646) der mehreren Andruckwalzen auf der zweiten Seite der tragenden Fläche (112) angeordnet ist und wobei die tragende Fläche (112) und die gewickelte Pflegeartikelstruktur (20) zwischen der wenigstens einen ersten Andruckwalze (412, 422, 612, 614, 622, 632, 642) und der wenigstens einen zweiten Andruckwalze (414, 424, 616, 626, 636, 646) angeordnet sind.

**6.** Vorrichtung (100, 400A, 400B) nach Anspruch 4 oder 5, wobei die tragende Fläche (112) eine biegsame tragende Fläche ist; oder wobei die tragende Fläche (112) eine im Wesentlichen nicht biegsame tragende Fläche ist; und/oder wobei die eine oder die mehreren Andruckwalzen nur auf der ersten Seite der tragenden Fläche (112) angeordnet sind.

**7.** Vorrichtung (100, 400A, 400B) nach einem der vorhergehenden Ansprüche, wobei eine oder mehrere Andruckwalzen der Druckanordnung (410, 420, 510, 520, 610, 620, 630, 640, 710, 720, 730, 740) für die wenigstens eine Lage (30, 50) Druck mit verschiedenen Laminierstufen bereitstellen.

**8.** Vorrichtung (100, 400A, 400B, 500A, 500B) nach einem der Ansprüche 4 bis 7, wobei die wenigstens eine erste Andruckwalze wenigstens eine Walze (612, 614, 642) mit konstantem Durchmesser umfasst, die einen im Wesentlichen konstanten Durchmesser aufweist, und/oder wobei die wenigstens eine erste Andruckwalze wenigstens eine Walze (622, 632) mit variablem Durchmesser umfasst, die unterschiedliche Durchmesser aufweist.

**9.** Vorrichtung (100, 400A, 400B, 500A, 500B) nach Anspruch 8, wobei die wenigstens eine Walze (622, 632) mit variablem Durchmesser wenigstens einen vorstehenden Abschnitt (622a, 622b, 632a, 632b) und wenigstens einen vertieften Abschnitt (622c, 632c) aufweist, wobei ein Durchmesser des wenigstens einen vorstehenden Abschnitts (622a, 622b, 632a, 632b) größer als ein Durchmesser des wenigstens einen vertieften Abschnitts (622c, 632c) ist.

**10.** Vorrichtung (100, 400A, 400B, 500A, 500B) nach Anspruch 9, wobei:

eine Breite des wenigstens einen vertieften Abschnitts (622c, 632c) in einer Richtung im Wesentlichen parallel zur Drehachse (623, 633) der wenigstens einen Walze (622, 632) mit variablem Durchmesser größer oder gleich einer Breite eines Abschnitts (13) der gewickelten Pflegeartikelstruktur (20) mit erhöhtem Belag ist; und/oder eine Tiefe des wenigstens einen vertieften Abschnitts (622c, 632c) in einer Richtung im Wesentlichen senkrecht zur Drehachse (623, 633) der wenigstens einen Walze (622, 632) mit variablem Durchmesser kleiner, gleich oder größer als eine Höhe des Abschnitts (13) der gewickelten Pflegeartikelstruktur (20) mit erhöhtem Belag ist; und/oder ein Bereich des wenigstens einen vertieften Abschnitts (622c, 632c) an die Form des Abschnitts (13) der gewickelten Pflegeartikelstruktur (20) mit erhöhtem Belag angepasst ist.

**11.** Vorrichtung (100, 400A, 400B, 500A, 500B) nach einem der Ansprüche 7 bis 9, wobei die Druckanordnung die wenigstens eine Walze (622) mit variablem Durchmesser und die wenigstens eine konstante Walze (642) umfasst, die längs eines Transportpfads der gewickelten Pflegeartikelstruktur (20) der Reihe nach angeordnet sind.

**12.** Vorrichtung (100, 400A, 400B, 500A, 500B) nach einem der vorhergehenden Ansprüche,

wobei die Delaminieranordnung (120) eine oder mehrere Delaminierwalzen (120a, 120b) oder eine oder mehrere Delaminierklingen umfasst; und/oder wobei die Delaminieranordnung (120) konfiguriert ist, die Schutzlage (32) von der gewickelten Pflegeartikelstruktur (20) zu delaminieren, indem eine Delaminierkraft ($F_{del}$) auf die Schutzlage (32) aufgebracht wird, wobei die Delaminierkraft ($F_{del}$) eine erste Delaminierkraftkomponente ($F_{del,y}$) in einer vertikalen Richtung (1) und eine zweite Delaminierkraftkomponente ($F_{del,x}$) in einer horizontalen Richtung (2) enthält und wobei die erste Delaminierkraftkomponente ($F_{del,y}$) kleiner als eine vertikale Kraftkomponente ($F_{belt,y}$; $F_{vac,y}$) ist, die durch die Transportvorrichtung (110) erzeugt wird und auf die gewickelte Pflegeartikelstruktur (20) in einer Richtung entgegengesetzt zur ersten Delaminierkraftkomponente ($F_{del,y}$) wirkt.

**13.** Vorrichtung (100, 400A, 400B, 500A, 500B) nach Anspruch 12, die ferner wenigstens eines der folgenden Elemente umfasst:

eine zweite Laminieranordnung (140), die konfiguriert ist, wenigstens eine weitere Lage (40) auf die gewickelte Pflegeartikelstruktur (20) zu laminieren, nachdem die Schutzlage (32) mittels der Delaminieranordnung (120) von der gewickelten Pflegeartikelstruktur (20) delaminiert worden ist; und/oder wenigstens eine Trennvorrichtung, die konfiguriert ist, die gewickelte Pflegeartikelstruktur (20) in mehrere gewickelte Pflegeartikel zu teilen.

**14.** Verfahren zum Laminieren wenigstens einer Lage (30, 50) auf eine gewickelte Pflegeartikelstruktur (20), wobei das Verfahren die folgenden Schritte umfasst:

Laminieren wenigstens einer Lage (30, 50) auf eine gewickelte Pflegeartikelstruktur (20), die auf einer tragenden Fläche (112) einer Transportvorrichtung (110) getragen wird;

Ausüben von Druck auf die wenigstens eine Lage (30, 50) unter Verwendung einer oder mehrerer Andruckwalzen, um die wenigstens eine Lage (30, 50), die wenigstens eine Schutzlage (32) aufweist, an der gewickelten Pflegeartikelstruktur (20) zu befestigen, während die gewickelte Pflegeartikelstruktur (20) auf der tragenden Fläche (112) der Transportvorrichtung (110) getragen wird, wobei der Abstand der einen oder der mehreren Andruckwalzen zur gewickelten Pflegeartikelstruktur (20) oder i zu einem Träger (11) der gewickelten Pflegeartikelstruktur (20) eingestellt werden kann; und Delaminieren der Schutzlage (32) von der gewickelten Pflegeartikelstruktur (20), die die wenigstens eine Lage (30, 50) aufweist, die darauf laminiert worden ist.

**Revendications**

1. Appareil (100, 400A, 400B, 500A, 500B) destiné à stratifier au moins une couche (30, 50) sur une structure d'article de soin de plaie (20), comportant :

   un dispositif de convoyage (110) ayant une surface de support (112) configurée pour supporter la structure d'article de soin de plaie (20) ;
   un premier agencement de stratification (130) configuré pour stratifier au moins une couche (30, 50) ayant au moins une couche protectrice (32) sur la structure d'article de soin de plaie (20) ;
   un agencement de pression (410, 420, 510, 520, 610, 620, 630, 640, 710, 720, 730, 740) ayant un ou plusieurs rouleaux de pression configurés pour appliquer une pression sur la au moins une couche (30, 50) stratifiée sur la structure d'article de soin de plaie (20) afin de fixer la au moins une couche (30, 50) sur la structure d'article de soin de plaie (20), dans lequel le ou les rouleaux de pression sont réglables dans leur distance par rapport à la structure d'article de soin de plaie (20) ou dans leur distance par rapport à un support (11) de la structure d'article de soin de plaie (20) ; et
   un agencement de délaminage (120) configuré pour délaminer la couche protectrice (32) de la structure d'article de soin de plaie (20) ayant la au moins une couche (30, 50) stratifiée sur celle-ci.

2. Appareil (100, 400A, 400B, 500A, 500B) selon la revendication 1, dans lequel la au moins une couche (30, 50) est une structure multicouche (30), l'agencement de pression (410, 420, 510, 520, 610, 620, 630, 640, 710, 720, 730, 740) est configuré pour appliquer la pression sur la structure multicouche (30) afin de presser une première couche (15) de la structure multicouche (30) contre la structure d'article de soin de plaie (20).

3. Appareil (100, 400A, 400B, 500A, 500B) selon la revendication 2, dans lequel les première et seconde couches (12, 15) sont des couches d'adhésif sensible à la pression.

4. Appareil (100, 400A, 400B, 500A, 500B) selon l'une quelconque des revendications précédentes, dans lequel la surface de support (112) inclut un premier côté configuré pour supporter la structure d'article de soin de plaie (20) et un second côté opposé au premier côté, et dans lequel au moins un premier rouleau de pression (412, 422, 612, 614, 622, 632, 642) parmi le ou les rouleaux de pression est situé sur le premier côté de la surface de support (112).

5. Appareil (100, 400A, 400B) selon la revendication 4, dans lequel le ou les rouleaux de pression sont une pluralité de rouleaux de pression, et au moins un second rouleau de pression (414, 424, 616, 626, 636, 646) de la pluralité de rouleaux de pression est situé sur le second côté de la surface de support (112), et dans lequel la surface de support (112) et la structure d'article de soin de plaie (20) sont intercalées entre le premier rouleau de pression (412, 422, 612, 614, 622, 632, 642) et le au moins un second rouleau de pression (414, 424, 616, 626, 636, 646).

6. Appareil (100, 400A, 400B) selon la revendication 4 ou 5, dans lequel la surface de support (112) est une surface de support souple ; ou la surface de support (112) est une surface de support sensiblement non souple ; et/ou le ou les rouleaux de pression sont situés uniquement sur le premier côté de la surface de support (112).

7. Appareil (100, 400A, 400B) selon l'une quelconque des revendications précédentes, dans lequel un ou plusieurs rouleaux de pression de l'agencement de pression (410, 420, 510, 520, 610, 620, 630, 640, 710, 720, 730, 740) exercent une pression sur la au moins une couche (30, 50) à différents niveaux de stratification.

8. Appareil (100, 400A, 400B, 500A, 500B) selon l'une quelconque des revendications 4 à 7, dans lequel le au moins un premier rouleau de pression inclut au moins un rouleau à diamètre constant (612, 614, 642) ayant un diamètre sensiblement constant et/ou le au moins un premier rouleau de pression inclut au moins un rouleau à diamètre variable (622, 632) ayant différents diamètres.

9. Appareil (100, 400A, 400B, 500A, 500B) selon la revendication 8, dans lequel le au moins un rouleau à diamètre variable (622, 632) a au moins une partie saillante (622a, 622b, 632a, 632b) et au moins une partie évidée (622c, 632c), dans lequel un diamètre de la au moins une partie saillante (622a, 622b, 632a, 632b) est plus grand qu'un diamètre de la au moins une partie évidée (622c, 632c).

10. Appareil (100, 400A, 400B, 500A, 500B) selon la revendication 9, dans lequel :

une largeur de la au moins une partie évidée (622c, 632c) dans une direction sensiblement parallèle à un axe de rotation (623, 633) du au moins un rouleau à diamètre variable (622, 632) est égale ou supérieure à une largeur d'une partie de tampon surélevée (13) de la structure d'article de soin de plaie (20) ; et/ou
une profondeur de la au moins une partie évidée (622c, 632c) dans une direction sensiblement perpendiculaire à l'axe de rotation (623, 633) du au moins un rouleau à diamètre variable (622, 632) est inférieure, égale ou supérieure à une hauteur de la partie de tampon surélevée (13) de la structure d'article de soin de plaie (20) ; et/ou
une zone de la au moins une partie évidée (622c, 632c) est adaptée à la forme de la partie de tampon surélevée (13) de la structure d'article de soin de plaie (20).

11. Appareil (100, 400A, 400B, 500A, 500B) selon l'une quelconque des revendications 7 à 9, dans lequel l'agencement de pression inclut le au moins un rouleau à diamètre variable (622) et le au moins un rouleau à diamètre constant (642) agencés en série le long d'un trajet de transport de la structure d'article de soin de plaie (20).

12. Appareil (100, 400A, 400B, 500A, 500B) selon l'une quelconque des revendications précédentes,

dans lequel le dispositif de délaminage (120) inclut un ou plusieurs rouleaux de délaminage (120a, 120b) ou une ou plusieurs lames de délaminage ; et/ou
dans lequel le dispositif de délaminage (120) est configuré pour délaminer la couche protectrice (32) de la structure d'article de soin de plaie (20) en appliquant une force de délaminage ($F_{del}$) à la couche protectrice (32),
dans lequel la force de délaminage ($F_{del}$) a une première composante de force de délaminage ($F_{del,y}$) dans une direction verticale (1) et une seconde composante de force de délaminage ($F_{del,x}$) dans une direction horizontale (2), et la première composante de force de délaminage ($F_{del,y}$) est plus petite qu'une composante de force verticale ($F_{Belt,y}$ ; $F_{vac,y}$) générée par le dispositif de convoyage (110) et agissant sur la structure d'article de soin de plaie (20) dans une direction opposée à la première composante de force de délaminage (F del,y).

13. Appareil (100, 400A, 400B, 500A, 500B) selon la revendication 12, incluant en outre au moins un élément parmi :

un second agencement de stratification (140) configuré pour stratifier au moins une couche supplémentaire (40) sur la structure d'article de soin de plaie (20) après que la couche protectrice (32) a été délaminée de la structure d'article de soin de plaie (20) au moyen de l'agencement de délaminage (120) ; et/ou
au moins un dispositif de séparation configuré pour séparer la structure d'article de soin de plaie (20) en une pluralité d'articles de soin de plaie.

14. Procédé pour stratifier au moins une couche (30, 50) sur une structure d'article de soin de plaie (20), comportant les étapes consistant à :

stratifier au moins une couche (30, 50) sur une structure d'article de soin de plaie (20) supportée sur une surface de support (112) d'un dispositif de convoyage (110) ;
appliquer une pression sur la au moins une couche (30, 50) en utilisant un ou plusieurs rouleaux de pression pour fixer la au moins une couche (30, 50) ayant au moins une couche protectrice (32) sur la structure d'article de soin de plaie (20) tandis que la structure d'article de soin de plaie (20) est supportée sur la surface de support (112) du dispositif de convoyage (110), dans lequel le ou les rouleaux de pression sont réglables dans leur distance par rapport à la structure d'article de soin de plaie (20) ou dans leur distance par rapport à un support (11) de la structure d'article de soin de plaie (20) ; et délaminer la couche protectrice (32) de la structure d'article de soin de plaie (20) ayant la au moins une couche (30, 50) stratifiée sur celle-ci.

# Fig. 1A

# Fig. 1B

# Fig. 1C

# Fig. 2

EP 4 091 591 B1

**Fig. 3**

EP 4 091 591 B1

# Fig. 4A

# Fig. 4B

## Fig. 5A

## Fig. 5B

**Fig. 6A**

50  13  613

610

612  614

11

116

617  616

1

2  3

**Fig. 6B**

622a  50  13  622c  622b

620

622

623

11

116

627  626

**Fig. 6C**

632a  50  13  632c  632b

630

632

633

11

116

637  636

**Fig. 6D**

50  13  643

640

642

11

116

647  646

**Fig. 7A**

710

612    50  13  613    614

11
116

**Fig. 7B**

622a  50  13  622c  622b    720

622
623

11
116

**Fig. 7C**

632a  50  13  632c  632b    730

632
633

11
116

**Fig. 7D**

50  13  643    740

642

11
116

## Fig. 8A

## Fig. 8B

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20170113972 A1